# EUROPEAN PATENT APPLICATION

(11) **EP 4 357 778 A1**
(43) Date of publication of application: **24.04.2024**
(21) Application number: 22202702.1
(22) Date of filing: 20.10.2022
(51) Int. Cl.: G01N 33/53, G01N 33/569, C12Q 1/04, C12Q 1/32, C12N 9/04, C12Q 1/00

(54) **TREATMENT OF MICROBIAL INFECTIONS DIAGNOSED USING THE BIOMARKER D-LACTATE**

(71) Applicant: Heraeus Medical GmbH, 61273 Wehrheim (DE)
(72) Inventor: Groth, Manuel, 61273 Wehrheim (DE)
(74) Representative: Heraeus IP

(57) **Abstract**

The present invention provides an antimicrobial agent for use in the treatment or prevention of a bacterial infection, wherein a patient is selected for treatment using the antimicrobial agent based on the level of D-lactate in a patient sample.

## Description

### FIELD OF THE INVENTION

The present invention relates to the field of diagnosing, treating, and preventing microbial infections.

### TECHNICAL BACKGROUND

In many cases, physicians at the point-of-care cannot immediately determine if a microbial infection is present in the patient. Instead, samples need to be sent to specialised laboratories, and are analysed with time-consuming methods that require expensive equipment and specialised personnel. The symptoms suffered by a patient having a microbial infection may be ambiguous, i.e., in many cases it is not clear whether an infection is indeed present. The anamnesis by a medical practitioner, as well as conventional clinical tests, such as cell count assays, may sometimes fail in diagnosing a microbial infection correctly. In some cases, different clinical laboratory parameters may apparently contradict each other.

Therefore, there is a need for new and improved diagnostic tests for microbial infections. This need in particular arises in indications where a potential microbial infection presents a severe risk to the patient, for example in the field of orthopaedic surgery. There is also a need to prevent the manifestation or exacerbation of microbial infections as early as possible.

Systems and methods for diagnosing bacterial infections are disclosed in, for example, EP2812700A1, EP3464645A1 and EP3322483A1.

### BRIEF SUMMARY OF THE INVENTION

It is an object of the present invention to provide improved systems, compounds and methods that address the problems of the prior art. For example, the present invention provides improved diagnostic methods that may result in more targeted treatment, and thus better therapeutic outcomes.

In the following, the present invention is illustrated by way of example, referring to preferred embodiments. Unless expressly noted otherwise or excluded based on the context, each of these embodiments can be combined with the features of the preceding embodiment(s). The present invention is not necessarily limited to these embodiments.

In a first embodiment, the present invention provides an antimicrobial agent for use in the treatment or prevention of a bacterial infection, wherein a patient is selected for treatment using the antimicrobial agent based on the level of D-lactate in a patient sample.

In a second embodiment, the patient is selected for treatment by further diagnosis of one or more further clinical parameters, wherein the further clinical parameter is preferably selected from the group consisting of cell culture, crystal formation, CRP, PCT, leukocyte count, granulocyte count, and polymorphonuclear cell count.

In a third embodiment, the patient has a history of a bacterial infection or arthropathic disease, wherein said arthropathic disease is preferably a non-infectious arthritis.

In a fourth embodiment, the patient shows ambiguous symptoms.

In a fifth embodiment, the bacterial infection is selected from the group consisting of a joint infection, meningitis and sepsis.

In a sixth embodiment, the joint infection is a native joint infection or a prosthetic joint infection.

In a seventh embodiment, the patient sample is pre-treated *in vitro* to remove contaminations before determining the level of D-lactate, wherein the sample is preferably pre-treated by filtration, centrifugation, and/or enzymatic catalysis.

In an eighth embodiment, the patient sample is selected from the group consisting of synovial fluid, cerebrospinal fluid, and blood.

In a nineth embodiment, determining the level of D-lactate comprises the use of an enzyme, wherein said enzyme is preferably selected from the group consisting of
(a) a protein having D-lactate oxidising activity that is independent of NAD and/or NADP,
(b) a protein having D-lactate oxidising activity isolated from a deltaproteobacterial species,
(c) a protein comprising SEQ ID NO: 1
(d) a protein comprising a sequence having at least 85%, 90% or at least 95% sequence identity to SEQ ID NO:1,
   and
(e) a functional fragment of (a), (b), (c) or (d).

In a tenth embodiment, determining the level of D-lactate is performed while maintaining a pH value of 7.5 to 9.5 in the sample.

In an eleventh embodiment, determining the level of D-lactate is performed using a dip stick test, a flow-through test, a colorimetric test, and/or an electrochemical test.

In a twelfth embodiment, the antimicrobial agent is administered locally, preferably by irrigation.

In a thirteenth embodiment, the antimicrobial agent is administered systemically, preferably intravenously or orally.

In a fourteenth embodiment, the treatment further comprises a surgical intervention, preferably the mechanical removal of bacteria, a bacterial biofilm and/or infected tissue.

In a fifteenth embodiment, the antimicrobial agent is administered for a duration determined by the level of D-lactate in said patient sample, preferably using repeated determination of the level of D-lactate.

Also disclosed herein is a method for selecting a patient for treatment with an antimicrobial agent, wherein a patient is selected for treatment using the antimicrobial agent based on the level of D-lactate in a patient sample, wherein said method is preferably a computer-implemented method.

### BRIEF DESCRIPTION OF THE DRAWINGS

Fig. 1 illustrates the purification and a photograph of an enzyme activity assay of D-lactate dehydrogenase from *Gluconobacter oxydans.*
Fig. 2 illustrates the purification and a photograph of an enzyme activity assay of D-lactate dehydrogenase from *Desulfovibrio vulgaris.*
Fig. 3 illustrates the results of an enzyme activity assay of D-lactate dehydrogenase from *Gluconobacter oxydans.*
Fig. 4 illustrates the results of an enzyme activity assay of D-lactate dehydrogenase from *Desulfovibrio vulgaris.*
Fig. 5 shows a calibration curve obtained with different D-lactate concentrations and D-lactate dehydrogenase from *Desulfovibrio vulgaris.*
Figs. 6A to 6E show a cut-off system in aqueous reactions.
Fig. 7 demonstrates a cut-off system in lateral flow reactions.
Fig. 8 shows lateral flow assays performed at different pH conditions in different buffer systems.

### DETAILED DESCRIPTION

The above and other aspects and embodiments of the invention will become clear from the further description herein. Unless indicated or defined otherwise, all terms used have their usual meaning in the art, which will be clear to the skilled person. Reference is for example made to the standard handbooks, such as Sambrook et al, "Molecular Cloning: A Laboratory Manual" (2nd Ed.), Vols. 1-3, Cold Spring Harbor Laboratory Press (1989); Lewin, "Genes IV", Oxford University Press, New York, (1990), as well as to the general background art cited herein.

Furthermore, unless indicated otherwise, all methods, steps, techniques and manipulations that are not specifically described in detail can be performed and have been performed in a manner known per se, as will be clear to the skilled person. Reference is for example again made to the standard handbooks, to the general background art referred to above and to the further references cited therein.

As used herein, the articles "a" and "an" refer to one or to more than one (e.g., to at least one) of the grammatical object of the article. When used herein, the term "comprising" and variations thereof such as "comprises" and "comprise" can be substituted with the term "containing" or "including" or "having" or "has", respectively, and vice versa.

Hence, with respect to each of the embodiments described herein whose components "have" or "comprise" a certain feature, such as a specific chemical or biological compound, another respective embodiment is contemplated wherein such component "consists of" said feature. Except explicitly noted otherwise, all features described herein are generally applicable to any of the aspects of the invention, i.e., the features of test systems can also be applied in any of the methods described herein, and vice versa.

In a first aspect, the present invention provides an antimicrobial agent for use in the treatment or prevention of a bacterial infection, wherein a patient is selected for treatment using the antimicrobial agent based on the level of D-lactate in a patient sample.

In some embodiments, the level of D-lactate in a patient sample is determined (i.e. measured) *in vitro.*

The term "antimicrobial agent" in the context of the present invention refers to a chemical substance which has the capacity to inhibit the growth of or to kill a microorganism. Different antimicrobial agents may have various mechanism of action, e.g. by destabilizing the microbial cell wall and/or cell membrane, by reacting with microbial cellular components such as nucleic acids, amino acids or lipids, by binding to bacterial ribosomal subunits, thereby inhibiting protein biosynthesis, inhibiting cell wall synthesis, e.g. by inhibiting peptidoglycan synthesis, interacting with the bacterial cytoplasmic membrane, thereby e.g. changing its permeability, inhibit bacterial DNA gyrase or topoisomerase IV enzyme, thereby inhibiting DNA replication and transcription, inhibiting folate synthesis, or inhibiting transcription by binding to RNA polymerase. The antimicrobial agent in the context of the present invention may for example be selected from the group comprising β-lactames, glycopeptides, polyketides, aminoglycoside antibiotics, polypeptide antibiotics, chinolones and sulfonamides. Preferably, the term refers to beta-lactam compounds like penicillines, cephalosporins or carbapenems; tetracyclines; macrolides; fluoroquinolones; sulfonamides; aminoglycosides; imidazoles; peptide-antibiotics and lincosamides. In some embodiments, the term "antimicrobial agent" relates to amoxicillin, flucloxacillin, penicillin G, ampicillin, methicillin, oxacillin, cefoxitin, ceftriaxone, ceftizoxime, imipenem, erythromicin, tylosin, tilmicosin, spiramycin, josamycin, azithromycin, clarithromycin, tetracycline, minocycline, doxycycline, lymecycline, norfloxacin, enoxacin, ofloxacin, co-trimoxazole, ciprofloxacin, trimethoprim, gentamicin, amikacin, metronidazole, bactiracin, clindamycin or lincomycin. In some embodiments, the term "antimicrobial agent" relates to ampicillin, cefotaxime, erythromycin, tetracycline, ciprofloxacin, co-trimoxazole, gentamicin, metronidazole, bacitracin or clindamycin.

Furthermore, an antimicrobial agent may comprise chlorhexidine, polyhexanide, iodine-povidone, activated zinc, sodium hypochlorite, or hypochlorous acid.

The antimicrobial agent preferably has a high selectivity for acting on microbes, i.e. it exerts a strong antimicrobial effect without substantially affecting viability of the patient's own cells, thus making the antimicrobial agent safe and effective for human or animal use according to medical regulatory standards. In some embodiments, the antimicrobial agent is a drug or active substance that has received a regulatory market authorization (e.g. FDA approval in the case of the USA) for human treatment before the priority date of this application in the territory where the present application has legal effect.

As used herein, the term "treat", "treating" or "treatment" in connection to a disease or disorder refers in some embodiments, to ameliorating the disease or disorder (i.e., slowing or arresting or reducing the development of the disease or at least one of the clinical symptoms thereof). In another embodiment "treat", "treating" or "treatment" refers to alleviating or ameliorating at least one physical parameter including those, which may not be discernible by the patient. In yet another embodiment, "treat", "treating" or "treatment" refers to modulating the disease or disorder, either physically, (e.g., stabilization of a discernible symptom), physiologically, (e.g., stabilization of a physical parameter), or both. In yet another embodiment, "treat", "treating" or "treatment" refers to preventing or delaying the onset or development or progression of the disease or disorder or a symptom thereof. As used herein, the term "subject" or "patient" refers to human and non-human mammals, including, but not limited to, primates, rabbits, pigs, horses, dogs, cats, sheep, and cows. In particular embodiments, a subject or patient is a human.

In some embodiments, the term "patient" or "subject" refers to a human being who is diseased with the condition (i.e., disease or disorder) described herein, such as a bacterial infection, and who would benefit from the treatment. As used herein, a subject is "in need of" a treatment if such subject (patient) would benefit biologically, medically or in quality of life from such treatment.

A "bacterial infection", as used herein, refers to a disease, disorder or medical condition which is at least partially caused by the presence of bacteria in the patient's body. A bacterial infection can be a local infection, which is localized to a single or multiple organs, or can be a systemic infection, which affects the entire body of the patient. Examples of a local infection include joint infections or meningitis. Examples of joint infections include bacterial infections of native joints, or infections of prostheses. A sepsis, as further described herein below, is a systemic bacterial infection. The term "bacterial infection" may also include a latent infection, wherein the patient may not yet exhibit any symptom of an infection, but may be at risk of developing a disease, disorder or medical condition due to the beginning growth of pathogenic bacteria in the patient's body.

Bacterial infections may be caused, for example, by *Staphylococcus aureus,* coagulase-negative staphylococci, streptococci, enteral bacteria, or other bacterial genera and species. Further examples of pathogens causing microbial infections include, without limitation, Neisseria gonorrhoeae and Pseudomonas aeruginosa.

An example of a joint infection is septic arthritis. Septic arthritis may be caused by microbial infection of a joint. Patients suffering from septic arthritis may have symptoms such as pain, fever, impaired movement, and swelling of the affected joint. However, similar symptoms may also occur due to an inflammation of the joint that is not caused by a bacterial infection.

Therefore, determining the level of D-lactate in a patient sample may serve as a guidance on whether the symptoms are indeed due to a bacterial infection. Similar considerations apply to other types of bacterial infections, such as meningitis or sepsis.

Sepsis is a systemic inflammatory response to a confirmed or suspected infection. As with many conditions the definition of sepsis has been updated over time. Sepsis may be defined as infection with at least 2 *Systemic Inflammatory Response Syndrome* (SIRS) criteria. Sepsis may also be defined as SIRS in the presence of a confirmed or suspected infection. A new definition of sepsis as a life-threatening organ dysfunction caused by a dysregulated host response to infection has been proposed (Singer et al. JAMA 2016; 315(8):801-810). The present invention is considered to be equally applicable to the evolving definitions of sepsis. The present invention is also applicable to identifying infection in a SIRS patient, irrespective of sepsis-related organ dysfunction assessment (SOFA) score. SIRS is an inflammatory state affecting the whole body. The four SIRS criteria are (Bone et al. Crit Care Med. 1992; 20(6)864-874):
- Temperature > 38°C (fever) or < 36°C (hypothermia)
- Heart rate > 90/min (tachycardia)
- Respiratory rate > 20/min or PaCO2 < 32 mm Hg (4.3 kPa) (tachypnea)
- White blood cell count > 12000/mm³ (leukocytosis) or < 4000/mm³ (leucopoenia); or > 10% immature neutrophils (bands).

When two or more of these criteria are met, patients may be diagnosed with SIRS.

The "level of D-lactate" refers to the concentration of D-lactate in a sample of a patient. Such sample can be, for example, a part of a tissue or body fluid derived from a patient. In one embodiment, a blood sample can be derived from a patient by use of a syringe. In a similar fashion, a sample of synovial fluid can be extracted from a patient's joint. In some embodiments, a tissue sample can be derived from a patient by surgical excision. Such tissue samples may need to be extracted and/or homogenized according to procedures known in the art before the level of D-lactate in the sample is determined.

In some embodiments, the level of D-lactate in the sample is determined *in vitro.* In some embodiments, the level of D-lactate is determined after the sample has been processed or pre-treated, as further described herein below.

In some embodiments, the patient is selected for treatment by further diagnosis of one or more further clinical parameters. As used herein, the term "clinical parameters" also includes biomarkers. In some embodiments, the further clinical parameter is selected from the group consisting of cell culture, crystal formation, CRP, PCT, leukocyte count, granulocyte count, and polymorphonuclear cell count. In some embodiments, the further clinical parameter is calprotectin, alpha-defensin or human neutrophil peptide 1-3.

For example, the patient can be selected for treatment based on the level of D-lactate and a further biomarker in the sample, such as CRP, PCT, calprotectin, alpha-defensin and/or human neutrophil peptide 1-3. In some embodiments, the patient is selected for treatment based on the level of D-lactate and CRP. In some embodiments, the patient is selected for treatment based on the level of D-lactate and PCT. In some embodiments, the patient is selected for treatment based on the level of D-lactate and calprotectin. In some embodiments, the patient is selected for treatment based on the level of D-lactate, PCT and CRP.

In contrast to other clinical parameters, the level of D-lactate may be especially useful to diagnose an infection that is due to a bacterial infection. For example, other clinical parameters may be influenced by an inflammatory condition that is not due to a bacterial infection.

The further clinical parameter may be determined from a sample of the patient. This can be a at the same sample that is used for determining the level of D-lactate, or it can be a different sample from the same patient. The further clinical parameter can be determined at the same time as the level of D-lactate, or at a different time.

In some embodiments, a given patient may have been subject to a diagnosis using one or more clinical parameters, but it may not yet be clear based on such diagnosis whether the patient has a bacterial infection, as further described herein in the context of the term "ambiguous symptoms". In such a case, the additional information provided by determining the level of D-lactate may provide additional guidance whether the patient has bacterial infection. In other embodiments, determining the level of D-lactate may be performed at the same time as the further clinical parameter, in order to provide a more accurate diagnosis as early as possible. Cell culture can be performed by subjecting a patient sample to suitable conditions in a specialized laboratory environment that allow the growth of pathogenic bacteria, i.e. bacteria causing bacterial infections.

Leukocyte count, granulocyte count, and polymorphonuclear cell count can be determined by subjecting a blood sample of a patient to analysis by specialized cell counting device, or manual analysis by microscopy. Crystal formation in a patient sample, for example in a synovial fluid sample, can be investigated by inspecting the sample using a microscope, preferably using a suitable contrast method (such as optical phase contrast or difference interference contrast). The level of biomarkers such as CRP (C-reactive protein) and PCT (procalcitonin) can be determined from a patient sample using bespoke commercially available assays and kits. Tests and methods for diagnosing a bacterial infection using PCT are disclosed in, without limitation, EP1121600B2, EP2028493B1, EP2084545B1, EP2174143B1, or EP2301626B1, which are hereby incorporated by reference in their entirety. Tests and methods for diagnosing a bacterial infection using CRP are disclosed in, without limitation, EP2335072B1, EP2647995B1, or EP1139101B1, which are hereby incorporated by reference in their entirety.

In another embodiment, the patient has a history of a bacterial infection or arthropathic disease. The arthropathic disease may be a non-infectious arthritis. A patient that previously suffered from bacterial infection or arthropathic disease may have an increased risk to develop another bacterial infection, for example a joint infection. This may happen, for example, when bacteria are transported in a patient's bloodstream from an existing site of infection to another part of the body. In some embodiments, the patient has a history of a hematogenic infection. Examples of hematogenic infections include, without limitation, skin, gum, teeth or urinary tract infections in which microbial pathogens are able to enter the bloodstream. In some embodiments, the patient may suffer from obesity, contaminated wounds, or diabetes, or may be a smoker, immunocompromised, aged at least 60, 70 or at least 80 years, or any combination thereof.

In a fourth embodiment, the patient shows ambiguous symptoms. The term "ambiguous symptoms", as used herein in the context of an infection, means that the patient may show one or more symptoms that are not sufficient to exclude that the symptoms may be caused by a non-infectious cause, such as an inflammation that is not originating from a bacterial infection. The term "ambiguous symptoms" may also include the absence of one or more typical infection symptoms. For example, the patient may have normal body temperature. The term "ambiguous symptoms" may include clinical parameters other than determining the level of D-lactate in a patient sample. In some embodiments, even after clinical investigation of standard laboratory tests, for example, one or more of leukocyte count, granulocyte count, polymorphonuclear cell count, biomarkers such as CRP and PCT, it may not be possible to unambiguously determine for the treating physician whether the patient symptoms actually originate from a bacterial infection or not. Therefore, according to some embodiments, the patient is additionally diagnosed based on the level of D-lactate as described herein. In some embodiments, the patient may have a negative radiological result, i.e., a radiological result that does not indicate an infection. In some embodiments, the patient may exhibit a swelling, redness of skin, pain, headache, fever, impaired mobility, and/or swollen lymph nodes. "Impaired mobility", as used herein, may refer to the inability to fully utilize a joint within the whole scope of a healthy degree of motion, or may refer to impaired walking ability.

In contrast to other biomarkers, the level of D-lactate may be especially useful to diagnose an infection that is due to a bacterial infection.

In some embodiments, the bacterial infection to be diagnosed using the methods described herein is selected from the group consisting of a joint infection, meningitis, and sepsis. A joint infection can be a native joint infection or a prosthetic joint infection. A native joint infection occurs on or in the vicinity of a patient's natural joint, whereas a prosthetic joint infection occurs on or in the vicinity of a prosthesis.

As used herein, the term "meningitis" refers to a bacterial infection that leads to an inflammation of the meninges, i.e., the protective membranes covering the brain and spinal cord. Meningitis is often associated with neck stiffness, high fever, mental impairments, and severe headache.

In some embodiments, the patient sample is pre-treated in order to remove contaminants before determining the level of D-lactate. Accordingly, the sample may e.g. be pre-treated by filtration, centrifugation, and/or enzymatic catalysis before the level of D-lactate is measured. For example, the sample may be filtered through a filter membrane having a suitable cut-off size to remove any proteins from the sample. Further details and means of pre-treatment of the sample are described herein below.

In some embodiments, the patient sample used for diagnosis by determining the level of D-lactate is selected from the group consisting of synovial fluid, cerebrospinal fluid, and blood. However, depending on the type of bacterial infection to be diagnosed, other sample types may be appropriate. In some embodiments, the level of D-lactate is determined from multiple different sample types derived from the same patient. For example, D-lactate may be determined in a synovial fluid sample and a blood sample of the same patient.

In some embodiments, determining the level of D-lactate comprises the use of an enzyme. This enzyme may have D-lactate oxidising activity. Such enzymes may also be termed as "D-lactate dehydrogenase" herein.

In some embodiments, the enzyme is selected from the group consisting of
(a) a protein having D-lactate oxidising activity that is independent of NAD and/or NADP,
(b) a protein having D-lactate oxidising activity isolated from a deltaproteobacterial species,
(c) a protein comprising SEQ ID NO: 1
(d) a protein comprising a sequence having at least 85%, 90% or at least 95% sequence identity to SEQ ID NO:1,
   and
(e) a functional fragment of (a), (b), (c) or (d).

The polypeptides of the invention may have a modified N-terminal sequence, e.g. a deletion of one or more of the N-terminal amino acids, or an exchange of e.g. the first, N-terminal amino acid (e.g. glutamate to alanine), to optimize the molecule for being expressed by using certain expression systems (such as specific vectors or host cells), or for being expressed as inclusion bodies or in soluble form, or for being secreted into the medium or the periplasmic space or for being contained within the cell, or for yielding a more homogenous product. The polypeptides of the invention may have a modified C-terminal sequence, such as an additional alanine, and/or further amino acid exchanges or deletions in the C-terminal part or at other defined positions within any of the framework regions, as explained e.g. in WO2012/175741, WO2011/075861, or WO2013/024059, in order to e.g. further enhance stability. An example of a polypeptide of the invention is an enzyme, more preferably a D-lactate dehydrogenase.

In the context of a nucleotide or amino acid sequence, the term "substantially identical" is used herein to refer to a first nucleic acid or amino acid sequence that contains a sufficient or minimum number of nucleotides or amino acids that are identical to aligned nucleotides or amino acids in a second nucleic acid or amino acid sequence such that the first and second sequences encode a polypeptide having common functional activity, or encode a common structural polypeptide domain or a common functional polypeptide activity. For example, nucleotide sequences having at least about 85%, 90%, 91 %, 92%, 93%, 94%, 95%, 96%, 97%, 98% or 99% identity to a reference sequence.

The terms "identical" or "percent identity," in the context of two or more nucleic acids or amino acid sequences, refer to two or more sequences or subsequences that are the same or have a specified percentage of nucleotides or amino acid residues that are the same, when compared and aligned for maximum correspondence. To determine the percent identity, the sequences are aligned for optimal comparison purposes (e.g., gaps can be introduced in the sequence of a first amino acid or nucleic acid sequence for optimal alignment with a second amino or nucleic acid sequence). The amino acid residues or nucleotides at corresponding amino acid positions or nucleotide positions are then compared. When a position in the first sequence is occupied by the same amino acid residue or nucleotide as the corresponding position in the second sequence, then the molecules are identical at that position. The percent identity between the two sequences is a function of the number of identical positions shared by the sequences (i.e., % identity=# of identical positions/total # of positions (e.g., overlapping positions x100). In some embodiments, the two sequences that are compared are the same length after gaps are introduced within the sequences, as appropriate (e.g., excluding additional sequence extending beyond the sequences being compared). For example, when variable region sequences are compared, the leader and/or constant domain sequences are not considered. For sequence comparisons between two sequences, a "corresponding" sequence motif refers to a sequence motif in the same location in both sequences (e.g., catalytically active center of each enzyme sequence).

The determination of percent identity or percent similarity between two sequences can be accomplished using a mathematical algorithm. A preferred, non-limiting example of a mathematical algorithm utilized for the comparison of two sequences is the algorithm of Karlin and Altschul, 1990, Proc. Natl. Acad. Sci. USA 87:2264-2268, modified as in Karlin and Altschul, 1993, Proc. Natl. Acad. Sci. USA 90:5873-5877. Such an algorithm is incorporated into the NBLAST and XBLAST programs of Altschul et al., 1990, J. Mol. Biol. 215:403- 410. BLAST nucleotide searches can be performed with the NBLAST program, score=100, wordlength=12, to obtain nucleotide sequences homologous to a nucleic acid encoding a protein of interest. BLAST protein searches can be performed with the XBLAST program, score=50, wordlength=3, to obtain amino acid sequences homologous to a protein of interest. To obtain gapped alignments for comparison purposes, Gapped BLAST can be utilized as described in Altschul et al., 1997, Nucleic Acids Res. 25:3389-3402. Alternatively, PSI-Blast can be used to perform an iterated search which detects distant relationships between molecules (Id.). When utilizing BLAST, Gapped BLAST, and PSI-Blast programs, the default parameters of the respective programs (e.g., XBLAST and NBLAST) can be used. Another preferred, non-limiting example of a mathematical algorithm utilized for the comparison of sequences is the algorithm of Myers and Miller, CABIOS (1989). Such an algorithm is incorporated into the ALIGN program (version 2.0) which is part of the GCG sequence alignment software package. When utilizing the ALIGN program for comparing amino acid sequences, a PAM120 weight residue table, a gap length penalty of 12, and a gap penalty of 4 can be used. Additional algorithms for sequence analysis are known in the art and include ADVANCE and ADAM as described in Torellis and Robotti, 1994, Comput. Appl. Biosci. 10:3-5; and FASTA described in Pearson and Lipman, 1988, Proc. Natl. Acad. Sci. USA 85:2444-8. Within FASTA, ktup is a control option that sets the sensitivity and speed of the search. If ktup=2, similar regions in the two sequences being compared are found by looking at pairs of aligned residues; if ktup=1 , single aligned amino acids are examined ktup can be set to 2 or 1 for protein sequences, or from 1 to 6 for DNA sequences. The default if ktup is not specified is 2 for proteins and 6 for DNA. Alternatively, protein sequence alignment may be carried out using the CLUSTAL W algorithm, as described by Higgins et al ., 1996, Methods Enzymol. 266:383-402.

Amino acid residues will be indicated according to the standard three-letter or one-letter amino acid code, as generally known and agreed upon in the art. When comparing two amino acid sequences, the term "amino acid difference" refers to insertions, deletions or substitutions of the indicated number of amino acid residues at a position of the reference sequence, compared to a second sequence. In case of substitution(s), such substitution(s) will preferably be conservative amino acid substitution(s), which means that an amino acid residue is replaced with another amino acid residue of similar chemical structure which has little or essentially no influence on the function, activity or other biological properties of the polypeptide. Such conservative amino acid substitutions are well known in the art, for example from WO1998/49185, wherein conservative amino acid substitutions preferably are substitutions in which one amino acid within the following groups (i) - (v) is substituted by another amino acid residue within the same group: (i) small aliphatic, nonpolar or slightly polar residues: Ala, Ser, Thr, Pro and Gly; (ii) polar, negatively charged residues and their (uncharged) amides: Asp, Asn, Glu and Gin; (iii) polar, positively charged residues: His, Arg and Lys; (iv) large aliphatic, nonpolar residues: Met, Leu, lie, Val and Cys; and (v) aromatic residues: Phe, Tyr and Trp. Particularly preferred conservative amino acid substitutions are as follows: Ala into Gly or into Ser; Arg into Lys; Asn into Gin or into His; Asp into Glu; Cys into Ser; Gin into Asn; Glu into Asp; Gly into Ala or into Pro; His into Asn or into Gin; lie into Leu or into Val; Leu into lie or into Val; Lys into Arg, into Gin or into Glu; Met into Leu, into Tyr or into lie; Phe into Met, into Leu or into Tyr; Ser into Thr; Thr into Ser; Trp into Tyr; Tyr into Trp or into Phe;

Val into lie or into Leu. The terms "polypeptide", "peptide" and "protein" (if single chain) are used interchangeably herein.

The terms "nucleic acid," "nucleic acid sequence," "nucleotide sequence," or "polynucleotide sequence," and "polynucleotide" are used interchangeably.

Preferably, the nucleic acid will be part of an expression vector, wherein said nucleic acid molecule is operably linked to at least one regulatory sequence, wherein such regulatory sequence may be a promoter, enhancer, or terminator sequence, and most preferably a heterologous promotor, enhancer, or terminator sequence.

In some embodiments, determining the level of D-lactate is performed while maintaining a pH value of 7.5 to 9.5 in the sample. When using the enzymes described herein, performing the measurement of D-lactate level at this pH can be beneficial, since the enzymatic reaction is performed with higher activity. In some embodiments, determining the level of D-lactate is performed while maintaining a different pH, such as a pH of about 6 or about 7.

The level of D-lactate can be determined using different types of tests, such as the test systems described herein. These tests may have different formats and detection principles. For example, determining the level of D-lactate may be performed using a dip stick test, or using a test in flow-through test format.

The test can be a colorimetric test, in which a D-lactate-oxidizing reaction of the enzyme can be coupled to the generation of a coloured substance (also known as "dye"), or a substance that can be detected using UV/Vis absorption spectroscopy.

It is also possible to use an electrochemical test, in which a D-lactate-oxidizing reaction of the enzyme can be coupled to the generation of an electrochemical potential. Such an electrochemical potential can be detected using a suitable detector, such as a current or voltage sensor. Such sensor can be, for example, a potentiometric sensor, such as an ISFET sensor. An electrical sensor may comprise a work electrode, a reference electrode and a counter electrode, respectively. The sensor may further comprise a data processing unit. Furthermore, the sensor may comprise a ground contact. Further details of useful tests are described herein, e.g. in the context of the detection of a reaction product of the enzyme.

The sensor may have a lower detection limit for D-lactate of below about 0.05 mM, more preferably below about 0.04 mM, 0.03 mM, 0.02 or 0.01 mM.

In some embodiments, the antimicrobial agent is administered locally. It can be advantageous to administer the antimicrobial agent at the site where an infection has been detected, e.g. the site where the sample has been derived from the patient, or at a site where an infection is suspected based on the diagnosis described herein.

The local administration may be performed by any suitable method. For example, the local administration can be performed by irrigation. Therefore, the antimicrobial agent can be administered in the form of a suitable irrigation solution, i.e., as a liquid formulation. Such a liquid formulation may further comprise a buffer component to control pH, or may be free of buffer components.

In some embodiments, the antimicrobial agent is administered systemically. For example, the antimicrobial agent may be administered intravenously or orally.

In some embodiments, the antimicrobial agent is administered both locally and systemically, either simultaneously or subsequently. For example, a first antimicrobial agent can be administered by irrigation of the infection site, and then a second antimicrobial agent can be administered systemically. In some embodiments, an antimicrobial agent that is administered systemically is an antibiotic. In some embodiments, an antimicrobial agent that is administered locally is an antimicrobial agent other than an antibiotic, such as chlorhexidine, polyhexanide, iodine-povidone, hypochlorous acid, and/or sodium hypochlorite.

The antimicrobial agent described herein can be formulated into various pharmaceutical compositions.

Administration of the antimicrobial agent or pharmaceutically acceptable forms thereof may be topical, i.e., the antimicrobial agent or a pharmaceutical composition thereof is applied directly where its action is desired (for example directly to a wound), or systemic. In turn, systemic administration can be enteral or oral, i.e., substance is given via the digestive tract, parenteral, i.e., substance is given by other routes than the digestive tract such as by injection or inhalation. Thus, the antimicrobial agent can be administered to a subject orally, parenterally, by inhalation, topically, rectally, nasally, buccally or via an implanted reservoir or by any other known method. The antimicrobial agents can also be administered by means of sustained release dosage forms.

For oral administration, the antimicrobial agent can be formulated into solid or liquid preparations, for example tablets, capsules, powders, solutions, suspensions and dispersions. The antimicrobial agent can be formulated with excipients such as, e.g., lactose, sucrose, corn starch, gelatine, potato starch, alginic acid and/or magnesium stearate.

For preparing solid compositions such as tablets and pills, an antimicrobial agent described herein can be mixed with a pharmaceutical excipient to form a solid preformulation composition. If desired, tablets may be sugar coated or enteric coated by standard techniques. The tablets or pills may be coated or otherwise compounded to provide a dosage form affording the advantage of prolonged action. For example, the tablet or pill can include an inner dosage and an outer dosage component, the latter being in the form of an envelope over the former. The two components can be separated by enteric layer, which serves to resist disintegration in the stomach and permit the inner component to pass intact into the duodenum or to be delayed in release. A variety of materials can be used for such enteric layers or coatings, such materials including a number of polymeric acids and mixtures of polymeric acids with such materials as shellac, cetyl alcohol, and cellulose acetate.

Compositions comprising an antimicrobial agent described herein may further comprise a pharmaceutically or physiologically acceptable carrier. Such carriers can be readily selected by one of ordinary skill in the art. Carriers for topical administration of the antimicrobial agents described herein include, but are not limited to, mineral oil, liquid petroleum, white petroleum, propylene glycol, polyoxyethylene and/or polyoxypropylene compounds, emulsifying wax, sorbitan monostearate, polysorbate 60, cetyl esters wax, cetearyl alcohol, 2-octyldodecanol, benzyl alcohol, and water. In formulating topical ointments, the antimicrobial agents described herein may be formulated in an oleaginous hydrocarbon base, an anhydrous absorption base, a water-in-oil absorption base, an oil-in-water water-removable base and/or a water-soluble base. The antimicrobial agents described herein may be formulated in an aqueous polymeric suspension including such carriers as dextrans, polyethylene glycols, polyvinylpyrrolidone, polysaccharide gels, Gelrite^{®}, cellulosic polymers like hydroxypropyl methylcellulose, and carboxy-containing polymers such as polymers or copolymers of acrylic acid, as well as other polymeric demulcents. The compositions comprising the antimicrobial agent described herein may be in any form suitable for topical application, including aqueous, aqueous-alcoholic or oily solutions, lotion or serum dispersions, aqueous, anhydrous or oily gels, emulsions obtained by dispersion of a fatty phase in an aqueous phase (O/W or oil in water) or, conversely, (W/O or water in oil), microemulsions or alternatively microcapsules, microparticles or lipid vesicle dispersions of ionic and/or nonionic type, creams, lotions, gels, foams (which will generally require a pressurized canister, a suitable applicator, an emulsifier and an inert propellant), essences, milks, suspensions, or patches. Topical compositions comprising the antimicrobial agent described herein may also contain adjuvants such as hydrophilic or lipophilic gelling agents, hydrophilic or lipophilic active agents, preserving agents, antioxidants, solvents, fragrances, fillers, sunscreens, odour-absorbers and dyestuffs.

In a further aspect, the topical compositions comprising the antimicrobial agent may be administered in conjunction with devices such as transdermal patches, dressings, pads, wraps, matrices and bandages capable of being adhered or otherwise associated with the skin or other tissue of a subject, being capable of delivering a therapeutically effective amount of one or more antimicrobial agents described herein.

In some embodiments, the treatment further comprises a surgical intervention. For example, such surgical intervention may comprise the mechanical removal of bacteria, a bacterial biofilm and/or infected tissue. In some embodiments, the mechanical removal of bacteria, a bacterial biofilm and/or infected tissue comprises a debridement, the use of an irrigation liquid, or the use of an oscillating, rotating and/or vibrating brush.

In some embodiments, the surgical intervention involves needle aspiration, arthroscopy, or arthrotomy. In some embodiments, the treatment further comprises support by dialysis, artificial respiration, drainage irrigation system, and/or a heart-lung machine.

The treatment using the antimicrobial agent can be continued for one week, two weeks, three weeks or four weeks. In some embodiments, the duration of the treatment using the antimicrobial agent can be determined by the level of D-lactate in a patient sample. This may involve, for example, determining the level of D-lactate in patient samples derived from the patient at different times during the course of the treatment. In some embodiments, the treatment using the antimicrobial agent is continued at least as long as the level of D-lactate exceeds a certain threshold as described herein, such as about 0.03 mM to 0.05 mM in a pre-treated sample, such as a synovial fluid, cerebrospinal fluid, or blood sample that may have been filtered to remove contaminants, such as protein components. Samples may be derived from the patient, for example, in intervals of one day, about three days or about seven days each, depending on the type of sample and the condition of the patient. In some embodiments, fresh samples are again analysed as soon as the patient presents himself with new or exacerbated symptoms. The samples can then be investigated for the level of D-lactate at each given point in time. In some embodiments, the patient is selected for treatment by further diagnosis of one or more further clinical parameters as described herein. In some embodiments, the level of D-lactate is determined (i.e. measured) *in vitro.*

In another aspect, the present invention provides a method of treatment of a bacterial infection, comprising administering an antimicrobial agent, wherein a patient is selected for treatment using the antimicrobial agent based on the level of D-lactate in a patient sample.

In this aspect, all embodiments described above for the aspect of an antimicrobial agent for use, and all embodiments described below for the aspect of a system for detecting a bacterial infection in a patient sample, are generally applicable accordingly.

According to a further aspect, the present invention provides a system for detecting a bacterial infection in a patient sample, which comprises a stereospecific enzyme having D-lactate oxidising activity, and a means for detecting a reaction product of said enzyme. The system can be used to determine the level of D-lactate in a sample of the patient as described herein, in order to diagnose a bacterial infection. In some embodiments, the level of D-lactate is determined (i.e. measured) *in vitro.*

The system may further comprise a sensor and/or a kit of reagents adapted for detection of D-lactate as described herein. The system may further comprise instructions for use that relate to the methods described herein.

An enzyme is a protein having catalytic activity. A protein is a polypeptide which may comprise amino acids that are selected from the group consisting of the 20 naturally occurring amino acids which occur e.g. in human beings, or may further comprise non-naturally occurring amino acids, such as synthetic derivatives of naturally occurring amino acids. Amino acids may have different stereo-configurations. In some embodiments, a protein described herein comprises only L amino acids. in other embodiments, a protein described herein comprises R amino acids. In some embodiments, a protein comprises both L and R amino acids.

A stereospecific enzyme is an enzyme that is stereoselective regarding its substance. For example, a stereospecific D-lactate dehydrogenase binds D-lactate with a much higher affinity than L-lactate. This difference can be an at least 10x, 100x or 1000x higher affinity between the two enantiomers of the substrates.

Deltaproteobacterial species can be any species of the class of deltaproteobacteria, being a class of gram-negative proteobacteria. All species of this class are, like all Proteobacteria, Gram-negative. Non-limiting examples of deltaproteobacteria are myxobacteria, Desulfovibrio, Desulfobacter, Desulfococcus, Desulfonema, Desulfuromonas, Geobacter, Pelobacter, Lawsonia and Syntrophus.

Deltaproteobacteria include the orders of Desulfarculales, Desulfobacterales, Desulfovibrionales, Desulfurellales, Desulfuromonadales, Myxobacteria and Syntrophobacterales. Desulfovibrionales include Desulfohalobiaceae, Desulfomicrobiaceae, Desulfonatronaceae and Desulfovibrionaceae.

The term "isolated," as used herein, refers to material that is removed from its original or native environment (e.g. the natural environment if it is naturally occurring). For example, a naturally occurring polynucleotide or polypeptide present in a living animal is not isolated, but the same polynucleotide or polypeptide, separated by human intervention from some or all of the coexisting materials in the natural system, is isolated. Such polynucleotides could be part of a vector and/or such polynucleotides or polypeptides could be part of a composition, and still be isolated in that such vector or composition is not part of the environment in which it is found in nature. For example, from a deltaproteobacterial species a protein having D-lactate oxidising activity of a deltaproteobacterial species can be expressed and subsequently purified from other cellular components.

"A protein having D-lactate oxidising activity isolated from a deltaproteobacterial species" shall also encompass, for example, a naturally occurring sequence from a deltaproteobacterial species that is expressed heterologously in a different organism, such as *E. coli* or *S. cerevisiae,* or another suitable expression system, and optionally subsequently purified.

An enzyme is a protein having catalytic activity. In many cases, enzymes have a high substrate specificity and selectivity, and therefore convert a specific chemical compound having a defined stereochemistry, or small defined group of such compounds, to a likewise well-defined product. In contrast, other chemically similar substrates are not converted, or with a much lower catalytic rate. An enzyme having "D-lactate oxidising activity", as used herein, means that said enzyme is capable of catalytically oxidizing D-lactate to pyruvate. In some embodiments, said D-lactate oxidising activity is dependent on the presence of an oxidizing cofactor, such as NAD, NADP or FAD. In some embodiments, the enzyme is substrate-specific D-lactate. In some embodiments the enzyme converts D-lactate with a turnover rate k_{cat} that is at least 5, 10, 20, 50, or at least 100 -fold higher than the turnover rate for any other substrate.

SEQ ID NO:1 corresponds to NCBI RefSeq accession No. WP_012612693.1, but not including the first amino acid (starting methionine), which is not expressed. The sequence of WP_012612693.1 is available online at www.ncbi.nlm.nih.gov/protein/WP 012612693.1/. SEQ ID NO:1 is therefore the following amino acid sequence in standard one-letter code:

PHTSDRSLPPTSDSAPRFVLTDAHRAAIVAAVGEAAVLHSPGEPYDRDASELRAPADLVVLPET
VEQVQALLRCASAHAIPVIPRGGGTGLAGGCLAVRGGVVLSLERMNRIRAIDTRNLVAEVEAGV
ISQRVRDAAAEQGLYYPPDPAGMDRSTIGGNVATNAGGPACVKYGVTRDYVLGVEAVLPDGE
LLRAGVRTRKGVVGYDMAHLLCGSEGTLGVITALTLKLVPLPPATVSMAVAFPDMAAAMRGVA
AVLGGGHLPSAIEFLDHRCIRLLGELLPIPVPGDKPSLLIIELDGAREQIVPELDLVAAICRQQGAT
HVLPAADEETRVRVWGARRQVSLRIHDYAALYMSEDVAVPLGAIAELVAALPEFEQRYGMEIFA

FGHAGDGNIHLNVTAPTRDTRDVVEQGIVELVGKVLELGGTISGEHGIGEAKKHLLPLELSPASI
RLQRGIRQVFDPRGIMNPGKVFGQ.

In one embodiment, the enzyme is a protein comprising a sequence having at least 85%, 90% or at least 95% sequence identity to SEQ ID NO:1. The enzyme can be a functional derivative or equivalent of a protein comprising a sequence of SEQ ID NO:1. A functional derivative or equivalent may be a natural derivative or is prepared synthetically. Exemplary functional derivatives include amino acid sequences having substitutions, deletions, or additions of one or more amino acids, provided that the biological activity of the protein is conserved, wherein the biological activity is in particular the D-lactate oxidising activity. The substituting amino acid desirably has chemico-physical properties which are similar to that of the substituted amino acid. Desirable similar chemico-physical properties include similarities in charge, bulkiness, hydrophobicity, hydrophilicity, and the like. For example, the present invention also contemplates mutations in the amino acid sequences according to the invention which may include substitutions, deletions, including internal deletions, additions, including additions yielding fusion proteins, or conservative substitutions of amino acid residues within and/or adjacent to the amino acid sequence, but that result in no functional ("silent") change, in that the change produces a functionally equivalent enzyme. Such change may e.g. comprise conservative amino acid substitutions which may be made on the basis of similarity in polarity, charge, solubility, hydrophobicity, hydrophilicity, and/or the amphipathic nature of the residues involved. For example, non-polar (hydrophobic) amino acids include alanine, leucine, isoleucine, valine, proline, phenylalanine, tryptophan, and methionine; polar neutral amino acids include glycine, serine, threonine, cysteine, tyrosine, asparagine, and glutamine, positively charged (basic) amino acids include arginine, lysine, and histidine; and negatively charged (acidic) amino acids include aspartic acid and glutamic acid.

In one example, the present invention relates to enzymes with enhanced expressibility, stability, or otherwise better suitability for diagnostic use, as well as altered/mutant derivatives thereof including, but not limited to ones exhibiting altered binding characteristics; e.g. altered association constants kₒₙ, dissociation constants k_{off}, and/or equilibrium constant or binding affinity, KD. The enzymes and methods disclosed herein encompass polypeptides and nucleic acids having the sequences specified, or sequences substantially identical or similar thereto, e.g., sequences at least 85%, 90%, 95% identical or higher to the sequence specified. In the context of an amino acid sequence, the term "substantially identical" is used herein to refer to a first amino acid that contains a sufficient or minimum number of amino acid residues that are i) identical to, or ii) conservative substitutions of aligned amino acid residues in a second amino acid sequence such that the first and second amino acid sequences can have a common structural domain and/or common functional activity. For example, amino acid sequences that contain a common structural domain having at least about 85%, 90%, 91 %, 92%, 93%, 94%, 95%, 96%, 97%, 98% or 99% identity to a reference sequence, e.g., a sequence provided herein SEQ ID NO:1.

Furthermore, the enzyme can also be a functional fragment of any of the sequences mentioned above. A functional fragment is any partial sequence, including N-terminal and/or C-terminal truncations of an amino acid sequence, as long as the original biological activity, in particular D-lactate oxidising activity, is maintained or substantially maintained. A functional fragment may, additionally or alternatively, have deletions with respect to any of the above-mentioned sequences, such as SEQ ID NO:1.

In some embodiments, the enzyme is an isolated protein, i.e. a purified enzyme that is substantially free from other cellular components of the host cell or system in which it has been expressed.

The system may further comprise a means for detecting a reaction product of the enzyme. This can be any product that is directly or indirectly produced by the action of the enzyme on D-lactate. For example, the system may comprise any means for detecting pyruvate and/or another product produced in the reaction of oxidizing D-lactate to pyruvate, such as NAD(P)H. The reaction product of said enzyme may also be a change in electrochemical potential in the sample, and/or a voltage or electrical current produced directly or indirectly by the action of the enzyme on the D-lactate in the sample. The reaction product of the enzyme may also be a cofactor or prosthetic group of the enzyme as further described below. For example, the enzyme may be a D-lactate dehydrogenase that has FAD as a prosthetic group. By oxidizing D-lactate, FAD is converted to FADH2, i.e. FADH2 is a "reaction product of the enzyme" as described herein. In this example, a means for detecting a reaction product of the enzyme may be a substance that is reduced by this FADH2 prosthetic group to yield a coloured reaction product ("dye").

If the reaction product is a change in electrochemical potential, the means for detecting a reaction product of the enzyme can be a detecting device or system that is adapted for detecting such change in electrochemical potential, i.e. an electrochemical sensor. Such a system may be adapted to reporting the change in electrochemical potential, which is generated by the enzyme based on the level of D-lactate in the sample, to a user of the system.

In some embodiments, the enzyme is NAD-independent, i.e. it can catalyse the reaction of D-lactate to pyruvate in the absence of NAD+. In some embodiments, the enzyme is NADP-independent. In some embodiments, the enzyme is NAD-independent and NADP-independent. Typically, commercially available D-lactate dehydrogenases are NAD-dependent. In some embodiments, the enzyme is a NAD-independent and NADP-independent D-lactate dehydrogenase. Instead of using NAD or NADP as a soluble cofactor, in some embodiments the enzyme uses a covalently bound cofactor such as a FAD prosthetic group that remains part of the enzyme during and after the catalytic reaction. This has the effect that the enzyme's reaction can more easily be coupled to other reactions, and is independent of soluble NAD or NADP in the sample.

The detection of a reaction product of the enzyme may occur by use of a colorimetric assay, an immunoassay or a sensor, such as an electrochemical sensor, including any suitable electrical detection method, or other suitable methods known in the field of diagnostics. A colorimetric assay typically uses a substance that is converted to a detectable reaction product whose concentration can be measured optically, for example by measuring the absorption of light of a predefined wavelength, or by observing a colour change that occurs when the reaction product of the enzyme is formed. For example, the enzyme may reduce a cofactor, such as FAD, to its reduced form, such as FADH2, while converting D-lactate to pyruvate. This reaction can be coupled to the reduction of a compound that is converted to a detectable substance. Preferably, the compound is directly converted to a detectable substance by the action of the enzyme. The "compound that is directly converted to a detectable substance" may also be referred to as a "dye" herein. This occurs, for example, if the enzyme comprises a covalently bound cofactor, also called prosthetic group, which in turn directly converts a compound to a detectable substance. Preferably, this cofactor is FAD or FADH2. The compound being converted to a detectable substance is preferably a dye, such as a tetrazolium dye. Examples of tetrazolium dyes include monotetrazolium and ditetrazolium dyes. Examples of monotetrazolium dyes include Thiazolyl blue tetrazolium bromide (MTT, CAS 298-93-1), Triphenyl tetrazolium chloride (TTC) or Tetrazolium Violet (TV). Examples of ditetrazolium dyes include Tetrazolium Blue Chloride (BTC), Nitrotetrazolium Blue Chloride (also called nitro-blue tetrazolium, NBT), and Tetranitroblue tetrazolium (TNBT). Tetrazolium dyes are described for example in Berridge et al, Biotechnology Annual Review 11, 2005, 127-152, which is hereby incorporated by reference in its entirety. In one embodiment, the compound that is directly converted to a detectable substance, also referred to as "dye", is MTT. Mono-tetrazolium dies, such as MTT, are preferred in some embodiments since they are exceptionally well soluble in aqueous solutions and also offer further advantages as described below. In one embodiment, the dye is converted into a non-water-soluble, or precipitable, product by the action of the enzyme.

As used herein, a "compound that is directly converted to a detectable substance by the action of the enzyme" means that the conversion of this compound is directly coupled to the enzymatic oxidation of D-lactate, rather than being converted indirectly by a reaction cascade. Such a test system is more robust since it is less likely to be influenced by other reactions that may occur in the sample during the test.

An immunoassay uses specific binding of an analyte as the detection principle. An example is an ELISA (enzyme linked immunosorbent assay) test.

In one embodiment, the enzyme is coupled to a solid support. The solid support can be a membrane, such as a cellulose-based membrane. In one example, the solid support is a nitrocellulose membrane. These membranes are convenient for immobilizing enzymes, since proteins are unspecifically absorbed on their surface. For other types of solid supports, such as a plastic surface, it may be preferable to coat the surface and/or derivatize the enzyme for coupling it to the solid support covalently. In one embodiment, the membrane is porous, such as a filter membrane.

Solid supports can be used for producing test strips. The enzyme coupled to a solid support can be used, for example, in a dip stick test. A dip stick is configured for testing a liquid sample by immersing the solid support in the sample, and subsequently removing it from the sample, thereby leaving behind a thin film of the sample on the enzyme-coated solid support that can be analyzed.

Another type of solid support is an electrode that can be used in electrochemical sensors as further described in more detail hereinbelow.

In one embodiment, the system is configured for the analysis of a sample in a flow-through format. For example, the enzyme may be coupled to a nitrocellulose filter membrane, and the system is configured to detect the presence of D-lactate in a sample that is delivered to the membrane and flows through this membrane.

The system according to the present invention may further comprise a cutoff reagent that is preferentially converted by the enzyme in comparison to a compound being converted to a detectable substance. In other words, the cutoff reagent will first be depleted before any positive readout signal is generated to a significant extent. In some cases, it may be desirable that the system only produces a detectable readout when a predetermined quantity or concentration of D-lactate in the sample is exceeded. In this case, the system may comprise a cutoff reagent that acts as a competing substrate in one of the involved reactions. For example, the cutoff reagent may be preferentially reduced in the presence of the enzyme in comparison to the compound being converted to a detectable substance as described above. In some embodiments, the cutoff reagent is suitable for accepting electrons from the enzyme or the enzyme's cofactor. Suitable cutoff reagents include Ellman's reagent, 1,4-benzoquinone and ferricyanide. A preferred cutoff reagent is a ferricyanide salt, such as potassium ferricyanide. By way of example, the system may comprise MTT as a compound that can be converted to a detectable substance, and potassium ferricyanide as a cutoff reagent. Virtually the complete amount of ferricyanide will be reduced by the enzyme reaction, before any MTT is converted. This may be the case even if MTT and ferricyanide are in contact with the enzyme and D-lactate simultaneously. It has been found that specific combinations of the compound that is directly converted to a detectable substance, or dyes, and cutoff reagents are especially preferred. In one embodiment, the cutoff reagent is ferricyanide, and the dye is a mono-tetrazolium dye, such as MTT. The use of cutoff reagents is further illustrated in the Examples herein.

The system may further comprise a buffering agent, which may preferably by configured to maintain a pH value of 7.5 to 9.5 during use. The inventors have found that test methods comprising preferable enzymes of the invention and other components of the assays of some embodiments described herein can preferably be carried out in a pH range of 7.5 to 9.5. In some embodiments, the buffering agent is configured to maintain a pH value of 7.5 to 9.0, 8.0 to 9.0, 7.5 to 8.0, or 7.5 to 8.5. The system of the invention preferably provides an increased buffer capacity in the above-mentioned pH ranges, such as in the range of 7.5 to 9.5. Buffer capacity is defined as the moles of H⁺ or OH⁻ necessary to change the pH of a solution by 1, divided by the pH change and the volume of buffer in liters; it is a unitless number. For example, the system may be configured to achieve a minimum buffer capacity of 0.001, 0.01 or 0.05 in the range of pH 7.5 to 8.5 at 25° C when the system is in use.

Useful buffering agents for use in the present invention are, for example, HEPES, HEPPS, NH₄Cl, bicarbonate, phosphate or Tris-HCI. In some embodiments, the buffering agent is Tris, preferably a Tris-HCI buffer adapted to maintain a pH in the range of pH 7.5 to 8.5 at 25° C. Tris is also known as tris(hydroxymethyl)aminomethane, CAS Nos. 77-86-1 (free base) and 1185-53-1 (hydrochloride). The use of Tris as a buffer may also be advantageous for its activity as a hydroxyl radical scavenger.

The system according to the invention may further comprise a means for controlling the sample volume. It is believed that the system may be more accurate if the sample amount and/or contact time with the reagents, such as the enzyme and/or means of detection, is precisely controlled. In some embodiments, the system comprises a well for applying a sample to the system, for example a well that is similar to plastic multi-well sample plates commonly used in biological applications and tests. The well may have a substantially cylindrical or conical shape. The well may comprise a mark in order to define the sample volume to be used in the system, similar to the marks of a measuring cylinder. The well may be configured to receive and hold the sample before and/or while the sample is measured.

In some embodiments, the system may further comprise means for controlling the sample volume flow through the system. Such means for controlling the sample volume flow may comprise, for example, a channel or reservoir that receives sample volume while and/or after it has been measured. The reservoir for receiving the sample after it has been measured can comprise a material can be configured to absorb the measured sample, such as a woven or nonwoven textile, foam or hygroscopic powder. For example, a cotton mesh or silica powder can be used to absorb the sample that has been measured.

The system according to the invention may further comprise means for removing contaminants from the sample before they are subjected to the enzyme reaction. Contaminants may be any part of the sample that is known or can be expected to interfere with the molecular mechanisms of the diagnostic test. For example, contaminants may interfere with the assay by specific or unspecific binding to any functional element of the diagnostic system. For example, if the system comprises a test strip membrane on which functional components are immobilized, such as, for example, a tetrazolium dye immobilised or a nitrocellulose membrane, contaminants may interfere with the assay by blocking the surface of said membrane.

Examples of contaminants are cells, and cellular components such as proteins and small organic molecules. Contaminants include alternative substrates of the enzyme (i.e. substrates other than D-lactate), inhibitors, proteases, and other enzymes that can react with D-lactate or other components of the system.

This means for removing contaminants may be adapted to removing contaminants by any suitable method, such as filtration, centrifugation, and/or enzymatic catalysis.

If the system is based on a colorimetric assay, contaminants may include substances that have a significant absorption at the wavelength that is used for the readout of the colorimetric assay, which is typically the global or a local absorption wavelength maximum of the dye used in the assay.

If the system comprises an electrochemical sensor, contaminants may include any substances that may either directly interfere with the reactions of this assay, or which may otherwise lead to a change in electrochemical potential in the sample under assay conditions.

In some cases, turbid samples may interfere with the diagnostic systems described herein, in particular in case of optical readouts.

By way of example, the system may comprise a filter as an integrated or separate component. The type of filter can be chosen based on the sample to be analysed. The pore size should be chosen so that any high molecular weight components are removed from the sample that may interfere with measurement. Suitable filter materials include, but are not limited to, PE, PP, PVDF, PTFE, cellulose or nylon. In case the contaminant material is prone to aggregate and clog the filter, a non-stick material like PTFE can be of particular advantage.

Suitable filters may have a size exclusion of, for example, 1, 5 or 10 kDa. In some embodiments, the filter has a size exclusion limit of 10 kDa.

The present inventors unexpectedly found that pre-filtering the sample is particularly helpful when the sample is synovial fluid. Pre-filtering may be helpful to remove components interfering with the enzymatic part of the detection, such as competing enzymes, or competing substrates, or inhibitors. Furthermore, pre-filtering may remove components that can interfere with the detection method used, e.g. components that absorb light or may generate or influence electrochemical potential.

Contaminants may also be removed by centrifugation, either in combination with a filter, or without using a filter. The sample may be filtered during, before, or after centrifugation to remove contaminants. Precipitation agents can be added to the sample to separate the contaminants more effectively by centrifugation. Precipitation agents include any suitable detergents, acids, bases, and salts that will denature contaminants or otherwise decrease solubility of the contaminants, but do not precipitate D-lactate. However, filtering may provide more reliable results than centrifugation alone, since the defined filter cut-off leads to a more precise exclusion based on the size of retained contaminants.

Alternatively or additionally, protein contaminants can be removed using a protease, such as trypsin, chymotrypsin, pepsin, or proteinase K. However, care should be taken that the protease will not inactivate the enzyme that is used for detection of D-lactate. Therefore, the protease should be removed, denatured and/or inhibited after the pre-treatment of the sample. For example, the sample may be briefly heated to a high temperature to denature the protease, or the protease may be removed by filtering and/or centrifugation as described above. Other types of contaminants may also be removed by action of enzymes or other catalysts.

In some embodiments the system is adapted for discriminating between a first concentration and a second concentration of D-lactate by a binary readout. A "binary readout" as used herein means that the system will provide a qualitative output in a "yes or no" fashion, in contrast to a quantitative output. In some applications, such as the diagnosis of a bacterial infection, it may be preferred to detect whether a pre-defined threshold concentration of D-lactate in the sample is exceeded. Accordingly, the system will only give a positive readout in case the predefined threshold concentration is exceeded. The first concentration of the lactate in the sample may for example be 1, 10, 20, 30, 40 or 45 µM D-lactate. The second concentration of the lactate in the sample may for example be 50, 55, 60 or 70 µM D-lactate. The first concentration can be lower than the second concentration. It is also preferred that the system is accurate and reproducible enough to reproducibly discriminate between the first and second concentration of D-lactate. For example, the system may be configured to discriminate between a concentration of a first concentration of 40 µM D-lactate and a second concentration of 60 µM D-lactate. In some embodiments, a cutoff reagent as described herein can be used to adapt the system for discriminating between a first concentration and a second concentration of D-lactate in a "yes-or-no" type fashion. In some embodiments, the amount of the enzyme, the sample volume to be measured and/or the amount of other reagents, such as a colorimetric dye, are chosen so that the system will only give a positive result in case the predefined threshold concentration of D-lactate in the sample is exceeded. This threshold may correspond to the first concentration, the second concentration, or a value in between the first and the second concentration.

In a further embodiment, the system comprises a sensor. The sensor can be an electrochemical sensor. Using such sensor, the presence of D-lactate is detected electrically, such as by a change in impedance or a change in conductimetric, amperometric, voltammetric or potentiometric signal, in the presence of the D-lactate. Thus, in an embodiment the D-lactate is detected by a change in electrical signal. Optionally, the concentration of D-lactate in the sample may be determined quantitatively by the electrical signal generated and can be used to determine whether a predefined threshold concentration, as described herein, is exceeded. Examples of suitable electrochemical sensors and methods are described in U. S. Patent Nos. 4,233, 029 to Columbus, 4,225,410 to Pace, 4,323,536 to Columbus, 4,008,448 to Muggli, 4,654,197 to Lilja et al., 5,108,564 to Szuminsky et al., 5,120,420 to Nankai et al., 5,128,015 to Szuminsky et al., 5,243,516 to White, 5,437,999 to Diebold et al., 5,288,636 to Pollmann et al., 5,628,890 to Carter et al., 5,682,884 to Hill et al., 5,727,548 to Hill et al., 5,997,817 to Crismore et al., 6,004,441 to Fujiwara et al., 4,919,770 to Priedel, et al., and 6,054,039 to Shieh.

The geometry of a sample can be controlled by a sample-receiving portion of a testing apparatus. For example, the sample can be placed onto a disposable test strip that can be inserted into a sensor device. The test strip may have a sample chamber (capillary fill space) to define the geometry of the sample. Alternatively, the effects of sample geometry may be limited by assuring an effectively infinite sample size. For example, the electrodes used for measuring the analyte may be spaced closely enough so that a drop of sample on the test strip extends substantially beyond the electrodes in all directions.

An electrochemical sensor as described herein may comprise one or more electrodes. Such electrodes may be coated with an enzyme having D-lactate oxidising activity as described herein. In some embodiments, the electrochemical sensor includes: a working electrode having a conductive material, and the enzyme arranged proximate to (e.g., disposed on) and in contact with the conductive material. One or more other electrode may be included, such as one or more counter electrodes, one or more reference electrodes and/or one or more counter/reference electrodes.

In some embodiments, the enzyme is positioned proximate to (e.g., disposed on) the surface of a working electrode of the electrochemical sensor. In some instances, a plurality of enzyme spots are positioned proximate to the surface of working electrode (e.g., in the form of spots). In certain cases, a discontinuous or continuous perimeter is formed around each of the plurality of enzyme spots are positioned proximate to the surface of the working electrode. Examples of depositing a plurality of reagent spots to the surface of an electrode as well as forming a discontinuous or continuous perimeter around each reagent spot is described in U.S. Patent Publication No. 2012/0150005 and in U.S. Patent Application No. 62/067,813.

The enzyme may be deposited onto the surface of the working electrode as one large application which covers the desired portion of the working electrode or in the form of an array of a plurality of enzyme spots, e.g., spaced apart from each other. Depending upon use, any or all of the enzyme spots in the array may be the same or different from one another. For example, an array may include two or more, 5 or more, 10 or more, 25 or more, 50 or more, 100 or more enzyme spots, or even 1000 or more, in an area of 100 mm² or less, such as 75 mm² or less, or 50 mm² or less, for instance 25 mm² or less, or 10 mm² or less, or 5 mm² or less, such as 2 mm² or less, or 1 mm² or less, 0.5 mm² or less, or 0.1 mm² or less.

The shape of deposited enzyme spot may vary within or between sensors. For example, in certain embodiments, the deposited membrane is circular. In other embodiments, the shape will be of a triangle, square, rectangle, circle, ellipse, or other regular or irregular polygonal shape (e.g., when viewed from above) as well as other two-dimensional shapes such as a circle, half circle or crescent shape. All or a portion of the electrode may be covered by the stabilized enzyme, such as 5% or more, such as 25% or more, such as 50% or more, such as 75% or more and including 90% or more. In certain instances, the entire electrode surface is covered by the enzyme composition (i.e., 100%).

Fabricating a sensor according to embodiments described herein may comprise producing a reproducible enzyme spot deposited on the surface of the electrode. For example, enzyme spots provided herein may deviate from each other by 5% or less, such as by 4% or less, such as by 3% or less, such as by 2% or less, such as by 1% or less and including by 0.5% or less. In certain embodiments, deposited enzyme spots show no deviation from one another and are identical.

In certain embodiments, methods further include drying an aqueous enzyme solution deposited on the electrode. Drying may be performed at room temperature, at an elevated temperature, as desired, such as at a temperature ranging from 25° C. to 100° C., such as from 30° C. to 80° C. and including from 40° C. to 60° C.

The enzyme can be mixed with a stabilizing agent, such as a sugar or a protein. Such stabilizing agent can prevent or decrease the denaturation of the enzyme, and may extend the storage duration (shelf life) of the system. An example for a sugar is trehalose. An example of a protein is albumin.

Examples of configurations for such sensors and methods for fabricating them may include, but are not limited to, those described in U.S. Pat. Nos. 6,175,752, 6,134,461, 6,579,690, 6,605,200, 6,605,201, 6,654,625, 6,746,582, 6,932,894, 7,090,756, 5,356,786, 6,560,471, 5,262,035, 6,881,551, 6,121,009, 6,071,391, 6,377,894, 6,600,997, 6,514,460, 5,820,551, 6,736,957, 6,503,381, 6,676,816, 6,514,718, 5,593,852, 6,284,478, 7,299,082, 7,811,231, 7,822,557 8,106,780, and 8,435,682; U.S. Patent Application Publication Nos. 2010/0198034, 2010/0324392, 2010/0326842, 2007/0095661, 2010/0213057, 2011/0120865, 2011/0124994, 2011/0124993, 2010/0213057, 2011/0213225, 2011/0126188, 2011/0256024, 2011/0257495, 2012/0157801, 2012/0245447, 2012/0157801, 2012/0323098, and 2013/0116524.

In certain embodiments, the working electrode and counter electrode of the electrochemical sensor as well as dielectric material of the sensor can be layered. For example, the sensor may include a non-conductive material layer, and a first conductive layer such as a conductive polymer, carbon, platinum-carbon, gold, etc., disposed on at least a portion of the non-conductive material layer. The conductive polymer may comprise PEDOT. The enzyme can be positioned on one or more surfaces of the working electrode, or may otherwise be directly or indirectly contacted to the working electrode. A first insulation layer, such as a first dielectric layer may disposed or layered on at least a portion of a first conductive layer and a second conductive layer may be positioned or stacked on top of at least a portion of a first insulation layer (or dielectric layer). The second conductive layer may be a reference electrode. A second insulation layer, such as a second dielectric layer may be positioned or layered on at least a portion of the second conductive layer. Further, a third conductive layer may be positioned on at least a portion of the second insulation layer and may be a counter electrode. Finally, a third insulation layer may be disposed or layered on at least a portion of the third conductive layer. In this manner, the sensor may be layered such that at least a portion of each of the conductive layers is separated by a respective insulation layer (for example, a dielectric layer).

In other embodiments, some or all of the electrodes may be provided in a co-planar manner such that two or more electrodes may be positioned on the same plane (e.g., side-by side (e.g., parallel) or angled relative to each other) on the material. For example, co-planar electrodes may include a suitable spacing there between and/or include a dielectric material or insulation material disposed between the conductive layers/electrodes. Furthermore, in certain embodiments one or more of the electrodes may be disposed on opposing sides of the non-conductive material. In such embodiments, electrical contact may be on the same or different sides of the non-conductive material. For example, an electrode may be on a first side and its respective contact may be on a second side, e.g., a trace connecting the electrode and the contact may traverse through the material. A via may provide an avenue through which an electrical trace is brought to an opposing side of a sensor.

A variety of approaches may be employed to determine the concentration of D-lactate with the electrochemical sensor. In certain aspects, an electrochemical D-lactate concentration monitoring approach is used. For example, monitoring the concentration of lactate using the sensor signal may be performed by coulometric, amperometric, voltammetric, potentiometric, or any other suitable electrochemical detection technique.

These methods may also be used in connection with a device that is used to detect and/or measure another analyte, including glucose, oxygen, carbon dioxide, electrolytes, L-lactate, host-specific biomarkers, or other moieties of interest, for example, or any combination thereof, found in a bodily fluid, including subcutaneous e.g., interstitial fluid, dermal fluid, synovial fluid, blood or other bodily fluid of interest or any combination thereof.

A host-specific biomarker, as used herein, denotes substances that are specifically produced or upregulated by a subject in response to a bacterial infection.

In certain embodiments, the method further includes contacting a sample with a sensor described herein, coupling conductive contacts of the electronics unit to contacts of the lactate sensor, collecting data using the electronics unit regarding a level of D-lactate from signals generated by the sensor, and forwarding the collected data from electronics unit to a receiver unit, e.g., by RF. The receiver unit may be a mobile telephone. The mobile telephone may include a software for use in detecting a bacterial infection. In certain embodiments, the sensor information is forwarded by RFID protocol, Bluetooth, and the like.

The sensor electronics unit may automatically forward data from the sensor/electronics unit to one or more receiver units. The sensor data may be communicated automatically and periodically, such as at a certain frequency as data is obtained or after a certain time period of sensor data stored in memory.

Aspects of the present disclosure include methods for fabricating an electrode having a D-lactate oxidizing enzyme for use in a sensor. Embodiments include forming an electrode and positioning the enzyme proximal to the electrode.

The sample to be used according to the invention can be any sample from a human or animal subject. The sample can be derived from any part of the body that is suspected of being subject to a bacterial infection. In some embodiments, the sample is derived from a body fluid. Examples of suitable samples are synovial fluid, cerebrospinal fluid, urine, and blood, respectively. Blood samples include whole blood, serum, and plasma samples. Such samples may be obtained by a clinician using a needle with a syringe attached. The needle may be inserted, optionally under radiological control, into one or more sites near the prosthesis under clinically sterile conditions, and fluid removed from the site using the syringe. For example, a needle may be inserted into the articular capsule or synovial cavity, and fluid is aspirated therefrom.

In some embodiments, the methods and systems according to the invention are designed to be practiced at the point of care, i.e., they do not require specialised technical equipment that is only present in clinical laboratories, but can be carried out directly by the practicing physician *in situ,* for example during a surgical intervention in an operating theatre. Point of care tests include tests comprising portable diagnostic devices.

In some embodiments, the methods and uses disclosed herein are carried out *in vitro,* i.e. they are not directly practiced on the human or animal body.

In another aspect, the present invention provides a method of diagnosing a bacterial infection of a patient, comprising subjecting a sample of the patient to be diagnosed to a diagnostic system as described herein, and a bacterial infection is diagnosed based on the detected level of D-lactate. In some embodiments, the sample is pretreated to remove contaminants therefrom, and subsequently subjected to a reaction with an enzyme described herein. In some embodiments, the patient is diagnosed using one or more further clinical parameters described herein, including biomarkers such as PCT, CRP or calprotectin.

In some embodiments, the level of D-lactate in the sample that is indicative of a bacterial infection is about 0.04 to 0.06 mM, for example 0.045 to 0.055 or 0.045 to 0.050 mM.

In some embodiments, the level of D-lactate in the sample that is indicative of a bacterial infection is about 0.02 to 0.05 mM, for example 0.025 to 0.045, or 0.030 to 0.040 mM.

In a further aspect, the present invention provides a method for selecting a patient for treatment with an antimicrobial agent, wherein a patient is selected for treatment using the antimicrobial agent based on the level of D-lactate in a patient sample. The method can be a computer-implemented method. The detected level of D-lactate in a sample of said patient can be obtained as described herein.

In a further aspect, the present invention provides a data carrier comprising means for carrying out a computer-implemented method of selecting a patient for treatment with an antimicrobial agent, wherein a patient is selected for treatment using the antimicrobial agent based on the level of D-lactate in a patient sample.

In a further aspect, the present invention provides a data processing apparatus for selecting a patient for treatment with an antimicrobial agent, wherein a patient is selected for treatment using the antimicrobial agent based on the level of D-lactate in a patient sample, wherein said apparatus is configured to carry out a computer-implemented method for selecting a patient for treatment with an antimicrobial agent as described above.

In a further aspect, the present invention provides a computer program comprising instructions which, when the program is executed by a computer, cause the computer to carry out a computer-implemented method for selecting a patient for treatment with an antimicrobial agent as described herein.

In a further aspect, the present invention provides a method for diagnosing a bacterial infection of a patient based on a detected level of D-lactate. The method can be a computer-implemented method. The detected level of D-lactate in a sample of said patient can be obtained as described herein. Based on the diagnosis, the patient can be selected for treatment with an antimicrobial agent as described herein.

In a further aspect, the present invention provides a data carrier comprising means for carrying out a computer-implemented method of diagnosing a bacterial infection of a patient based on a detected level of D-lactate as described herein.

In a further aspect, the present invention provides a data processing apparatus for diagnosing a bacterial infection of a patient based on a detected level of D-lactate in a sample of said patient, wherein said apparatus is configured to carry out a computer-implemented method as described above.

In a further aspect, the present invention provides a computer program comprising instructions which, when the program is executed by a computer, cause the computer to carry out a computer-implemented method of diagnosing a bacterial infection of a patient based on a detected level of D-lactate as described herein.

The invention is further illustrated by the following examples, which should be construed as exemplary and non-limiting.

### EXAMPLES

### Example 1: Recombinant Production of D-lactate dehydogenases.

The amino acid sequences for expression of D-lactate dehydogenases were obtained from accession numbers WP_02471710 (*Gluconobacter oxydans,* Sheng et al (2015) Appl Environ Microbiol 81:4098 - 4110) and WP_012612693 (*Desulfovibrio vulgaris;* SEQ ID NO:1; Ogata et al (1981) J. Biochem 89, 1423-1431; Vita et al (2015) Front Microbiol. 6, 606), and WP_014015113 (*Megasphaera elsdenii*; Olson and Massey (1979) Biochemistry 18(21):4714-24), respectively.

The nucleic acid sequence was derived based on *E. coli* codon usage. Synthesis and cloning of the protein genes into a pET system vector (Thermo Fisher) was then performed and the recombinant vectors were used to transform an expression strain of *E. coli* in order to commence recombinant protein production.

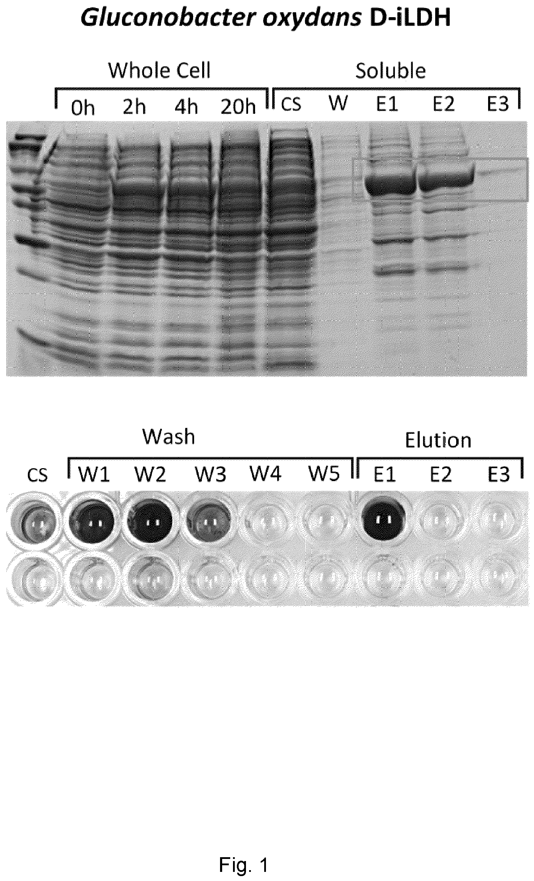

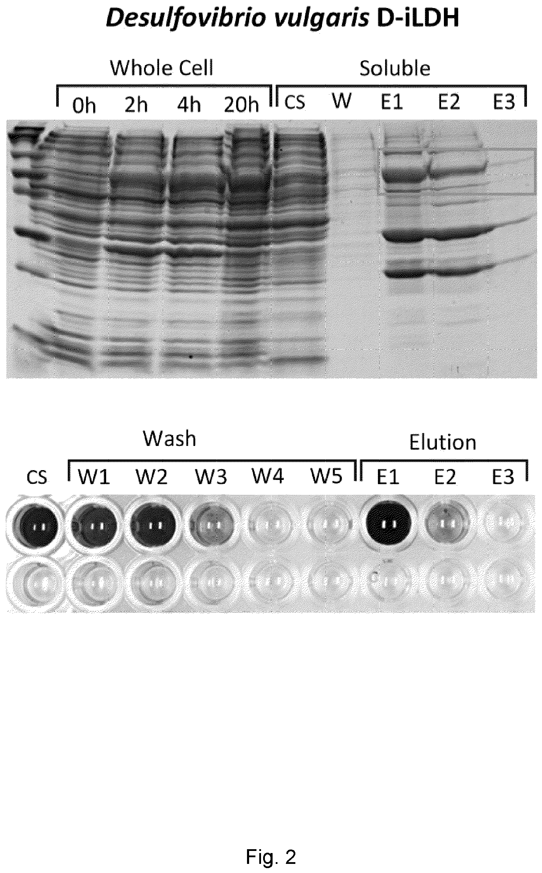

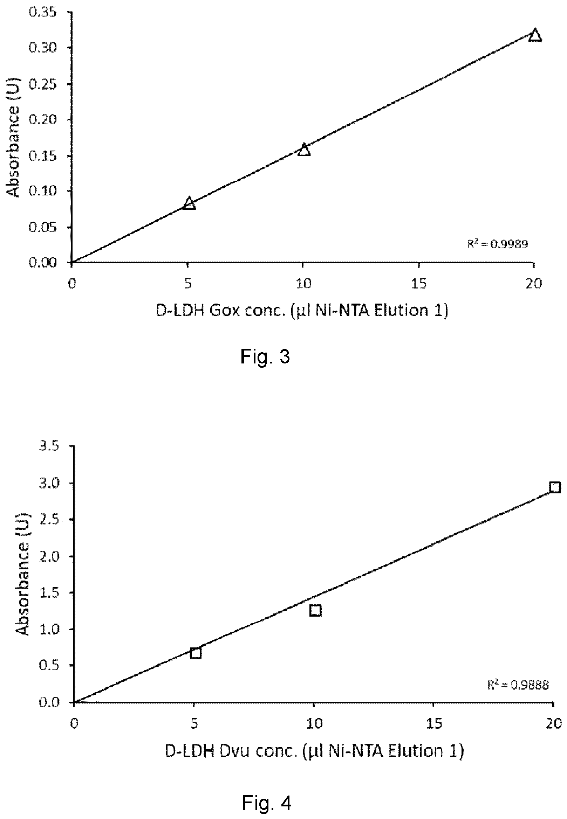

Recombinant expression was performed at 37°C in 50ml LB media using pET- 100/D-Topo in *E. coli* BL21 Star (DE3) with an N-terminal His tag. Induction was performed by addition of 1 mM IPTG at A600nm = 0.6. Purification of recombinant protein in the cell soluble extract (CS) after 20 hours prepared in a total volume of 3ml using BPER with HALT DNAse and Lysozyme, was performed by Ni-NTA column following standard protocols with elution in 250mM imidazole. Wash and elution fractions from the column were analysed on the SDS PAGE and in an activity assay as indicated. Expression of the recombinant protein from both enzymes was visible on the gel in elution samples from the Ni-NTA column at the predicted subunit size of D-iLDH of about 50 kDa. Very significant levels of active enzyme were observed in the CS samples. Whilst some enzyme was detected in the wash fractions indicating the column was overloaded, the recombinant protein in each case was also present in the elution fractions indicating correct interaction of the His tag with the column. The activity of each purified enzyme was found to be proportional to the amount of the enzyme. The specific activity of each sample was determined using enzyme and protein assays. The first eluted enzyme fraction for *G*. *oxydans* was found to be 180 U/mg and for *D*. *vulgaris* was 1506 U/mg corresponding to an enrichment of approximately 7-fold as a result of the His column purification. For the activity assay, enzyme samples (0-20 µl) were incubated with D-lactate (4 mM), MTT (2 mM), FAD (0.1 mM), Tris-HCI (50 mM), pH 8.5. OD570 was measured for 10 min at 20°C. Protein concentrations were determined using Qubit assay kit. The results of the activity assay are shown in Fig. 1 (lower portion) and Fig. 3 for the *G*. *oxydans* enzyme and in Fig. 2 (lower portion) and Fig. 4 for the *D. vulgaris* enzyme, respectively.

The *M. elsdenii* enzyme only produced a low level of expression, did not bind to the affinity column and was not further investigated for this reason. The *D*. *vulgaris* enzyme exhibited the highest expression yield and activity. A calibration graph was produced using the purified recombinant enzyme in order to determine the potential for the quantitative detection of D-Lactate. This calibration graph revealed a linear range until the MTT electron acceptor became limiting, evidencing the rapid rate of reaction with this enzyme. The activity level in various different buffers was observed over several days with no significant loss of activity. Of the three tested enzymes, the *D*. *vulgaris* enzyme exhibited the highest expression yield and also highest enzyme activity.

### Example 2: Calibration assay

In this assay, the recombinant *D*. *vulgaris* enzyme obtained as described in Example 1 was incubated with different D-lactate concentrations to generate a calibration curve.

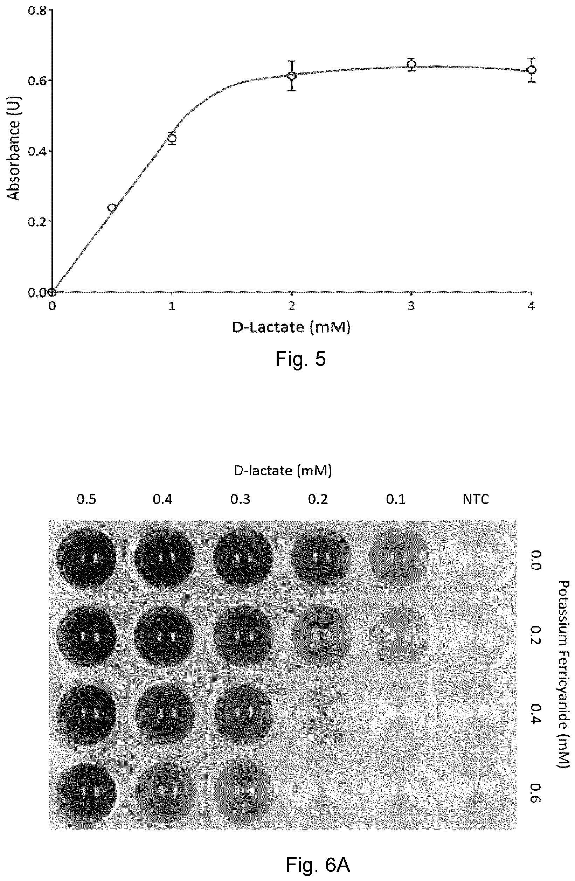

A linear end-point absorbance was observed up to between 1 - 2 mM D-lactate providing for accurate quantitation in the lower concentration range. The reaction mix contained D-lactate (variable), *D*. *vulgaris* enzyme prepared according to Example 1, MTT (2 mM), FAD (0.1 mM), Tris-HCI (50 mM) pH 8.5. Absorbance at 570 nm was measured regularly over 20 min at 20°C. To determine an accurate endpoint read, samples were diluted 10-fold and A570 measured after more than 20 min. The results are shown in Fig. 5.

### Example 3: Cut-off reagent evaluation

Given that D-Lactate is a quantitative biomarker with a defined clinically significant threshold within synovial fluid, it is desirable that an accurate "cut-off" can be integrated into the assay in order to achieve the most straight-forward product format to provide a "yes-or-no" -type test, i.e. a binary readout. An alternative approach would be to develop a semi-quantitative assay that relies on the user to approximate the level of D-Lactate and determine the clinical significance of the result. Various theoretical approaches were considered and tested but found not to be effective, including using tetrazolium dyes and using a combination of two different D-lactate dehydrogenase enzymes. The preferred approach that was found to work was to use competitive secondary substrates that remove assay colour signal development below the defined threshold by accepting electrons from the flavin nucleotide bound within the D-lactate dehydrogenase enzyme.

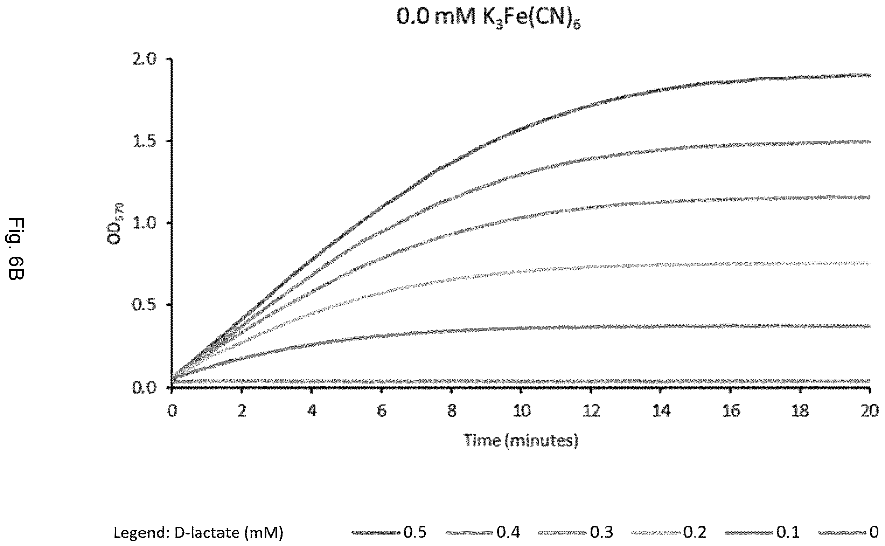

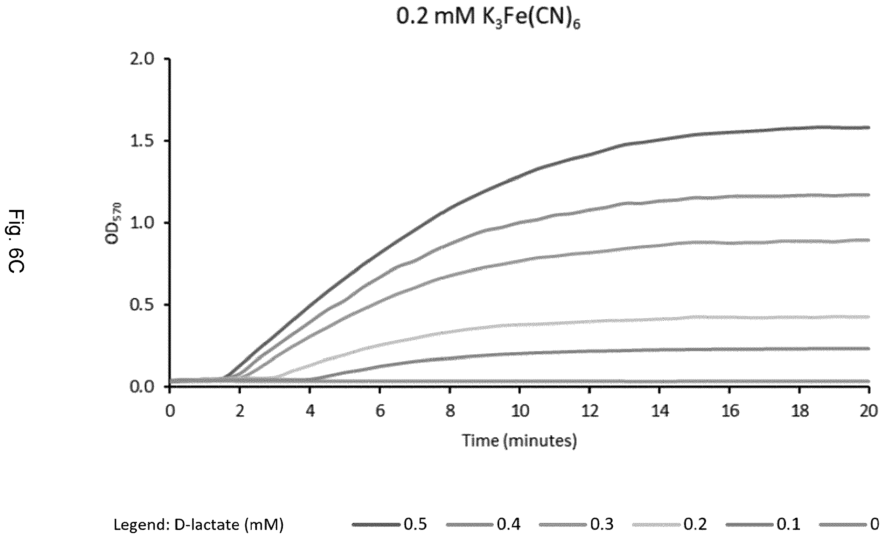

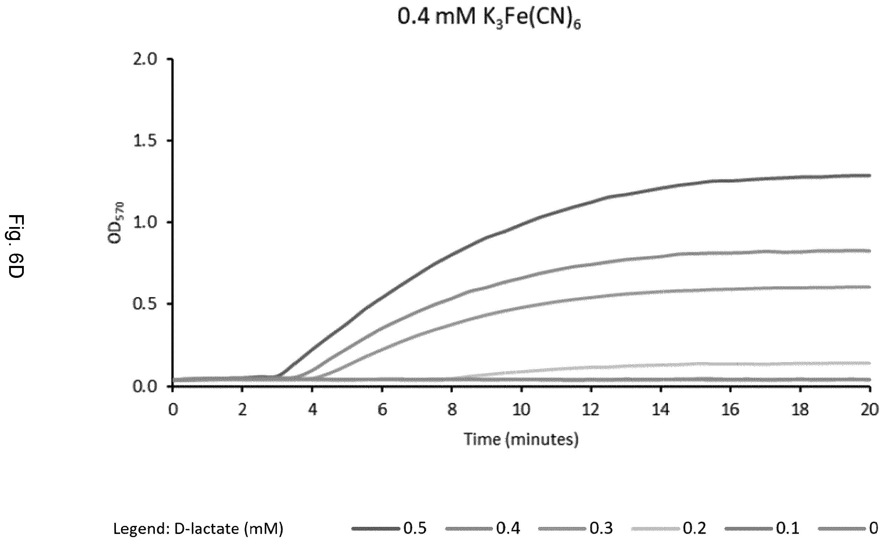

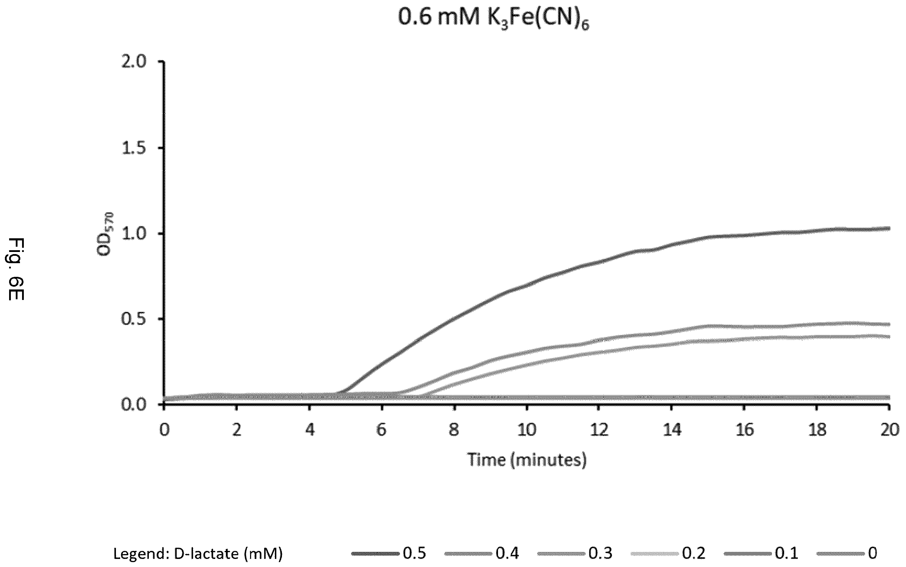

Three potential cutoff reagents were tested, Ellman's reagent, 1,4-benzoquinone and potassium ferricyanide. Of these both benzoquinone and ferricyanide were found to function effectively with D-lactate dehydrogenase enzymes, and ferricyanide only was found to function effectively with NAD(P)-independent D-lactate dehydrogenase enzymes. Selected data demonstrating the proof-of-concept of "cut-off' engineering for both enzymes is displayed in Figures 6 to 9 and further described below. The tests were performed in solution and on lateral flow test strips. The test strips were made by immobilizing the MTT dye on nitrocellulose membranes.

Figures 6A to 6E show a cut-off system in aqueous reactions. Assays were carried out in aqueous buffer, in a 96-well plate. Reactions were at RT with 100 mM Tris-HCI pH 8.0, MTT 1 mM and D-lactate dilutions from 0.5 mM to 0.1 mM including a no target control (NTC). Potassium ferricyanide was added at 0.2 mM, 0.4 mM or 0.6 mM. Optical density was measured at OD570 for 20 minutes. The results show a shift in OD570 and time correlating with the increased concentration of ferricyanide, and the time taken to reduce the ferricyanide with the given assay. This demonstrates successful use of ferricyanide as a cut-off reagent and shows it out-competes D-lactate as a competitive inhibitor with D-iLDH in these conditions.

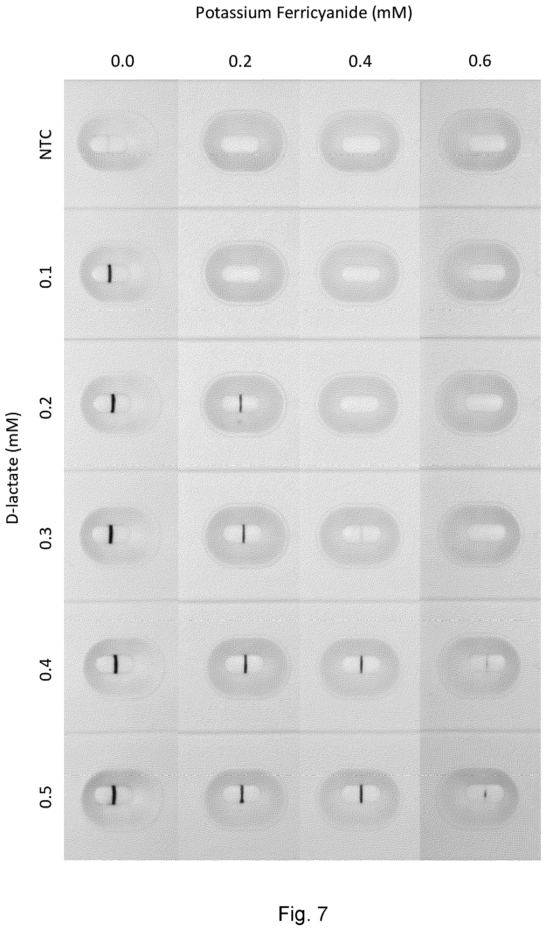

Figure 7 demonstrates a cut-off system in lateral flow reactions. Test strips were printed (1 µl cm-1) with D-iLDH 2400 U ml-1, H2O. Strips were made using Amersham Protran Supported 0.45 µm nitrocellulose. Assays were performed at RT with 100 mM Tris-HCI pH 8.0, MTT 1 mM and D-lactate dilutions from 0.5 mM to 0.1 mM including a no target control (NTC). Potassium ferricyanide was added at 0.2 mM, 0.4 mM or 0.6 mM. The results show a shift in D-lactate detection limit correlating with the increased concentration of ferricyanide.

### Example 4: Buffer and pH optimisation.

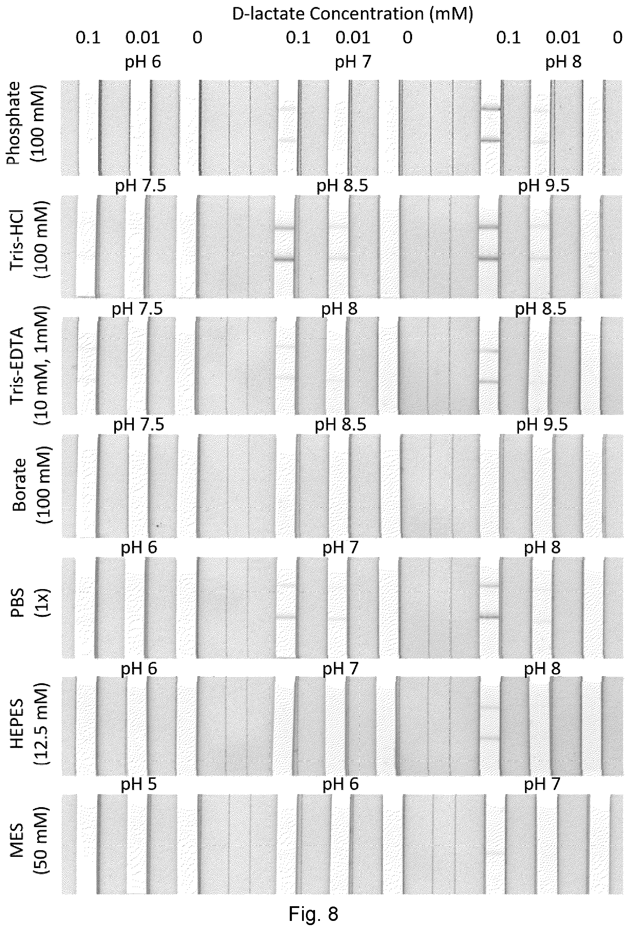

Seven different reaction buffers were tested at varied concentrations and at different pHs in the test strip assay format according to Example 3. Optimum pH for the D-iLDH assay strip was found to be between pH 8 and pH 9; very little enzyme activity was found ≤ pH 7. Tris-HCI pH 8.5 was considered as best buffer deemed by signal intensity and development time. Reaction mix: D-lactate (variable), NBT (0.3 mM), buffer (variable). Strips were each printed with two identical enzyme lines and observed during development; photographs taken once the strip was dry (20 min). The results are shown in Figure 8.

### Example 5: Clinical sample evaluation and sample pre-treatment.

Synovial fluid samples were retrieved from clinical patients suspected of having a periprosthetic joint infection. The samples included 86 samples from aseptic joint failures (68 knee and 18 hip samples) and 34 samples from prosthetic joint infections (20 knee and 14 hip samples).

Synovial fluid was aspirated under sterile conditions preoperatively in the outpatient department or during revision surgery before opening the joint capsule. One ml of synovial fluid was inoculated into a pediatric blood culture bottle (BacTec PedsPlus/F, Beckton Dickinson and Co), 1 ml was introduced in a native vial for aerobic and anaerobic culture (0.1 ml each) and the remaining fluid was inoculated in thioglycolate broth for enrichment. The pediatric blood culture bottle was incubated at 36 ± 1 °C for 14 days or until growth was detected. The aerobic cultures were incubated at 37 °C and inspected daily for 7 days, and the anaerobic ones were incubated for 14 days. The colonies of microorganism morphology were identified by standard microbiological methods using automated system VITEK 2 (bioMérieux, Marcy L'Etoile, France). For detection of urate and pyrophosphate crystals, a 1 ml-aliquot was sent to the pathologist for examination of the synovial fluid with polarization microscopy. In addition, 3-5 periprosthetic tissue samples were collected during surgery from the implant-bone or cement-bone interface for microbiological and histopathological analysis, if revision surgery was performed. Periprosthetic tissue culture was considered positive if a high-virulent organism grew in ≥1 specimen of synovial fluid, periprosthetic tissue or sonication (Staphylococcus aureus, Enterobacteriaceae, Streptococcus spp., Candida spp.) or a medium or low-virulent organism grew in ≥2 specimen (coagulase-negative staphylococci, enterococci, Cutibacterium [formerly known as Propionibacterium ] spp., and other bacteria of the skin microbiome). The retrieved prosthetic components were sent for sonication. Sonication was considered positive if ≥1 CFU/ml of a high-virulent organism or > 50 CFU/ml of a low-virulent organism grew in sonication fluid.

The D-lactate concentration of each synovial fluid sample (50 µl) was determined using commercial D-lactate kits from Sigma-Aldrich (MO, USA), incubation time: 30 min at room temperature, and a microplate photometer (λ = 450 nm).

Since the first series of tests did not meet the expectations, an additional pre-filtering step was included in the test protocol to improve the sample preparation. The samples were measured with and without such a filtration step in order to evaluate the effect of this sample pretreatment. Filtration was performed by centrifugation using a 10kDa filter at 4°C for 60 min. (Microcon-10kDa Centrifugal Filter Unit with Ultracel-10 membrane, Millipore). These results confirmed that the pre-filtering significantly improved sensitivity and specificity of the test in diagnosing a periprosthetic joint infection. The results were confirmed by leukocyte count, cell culture and histopathology. The leukocyte count was determined by flow cytometry using an automated haematology analyzer (XE-2100, Sysmex, Norderstedt, Germany).

Youden's J statistic was used for determining D-lactate cut-off point on the ROC curve. The area under the ROC curve (AUC) was used to assess the diagnostic performance of D-lactate test, leukocyte count cell culture and histopathology. Two-sided independent samples Student's t -test was applied to assess statistical significance in the mean concentration of D-lactate between groups. For all statistical analyses IBM SPSS 22.0 (Statistical package for the Social Sciences Corporation, Chicago, IL, USA) was used.

The results are shown in Table 1 below. In conclusion, the tests confirmed that, compared to other established test methods, the determination of the D-lactate concentration in pre-filtered synovial fluid samples is the most sensitive and specific diagnostic option for prosthetic joint infection. For filtered samples, a cutoff value of 0.0475 mmol/L D-lactate was determined as the best threshold concentration to achieve an optimal result for both sensitivity and specificity.

**Table 1: Results from clinical samples of Example 5.**

| PPV: Positive predictive value; NPV: Negative predictive value; LLR+: Likelihood ratio positive; LLR-: Likelihood ratio negative; AUC: Area-under-the-curve from specificity vs. sensitivity plot | | | | | | | |
|---|---|---|---|---|---|---|---|
| **Tests** | **Sensitivity, %** | **Specificity, %** | **PPV, %** | **NPV, %** | **LLR+** | **LLR-** | **AUC** |
| D-lactate, mmol/L | 94.12 | 88.37 | 76.19 | 97.44 | 8.09 | 0.07 | 0.95 |
| D-lactate (unfiltered), mmol/L | 87.50 | 74.42 | 56 | 94.12 | 3.42 | 0.17 | 0.88 |
| Leukocytes, cell/µl | 88.0 | 88.75 | 70.97 | 95.95 | 7.82 | 0.14 | 0.92 |
| Culture | 70.59 | 89.53 | 72.73 | 88.51 | 6.75 | 0.33 | - |
| Histopathology | 65.0 | 100 | 100 | 80.0 | - | 0.35 | - |

### Example 6: Clinical sample evaluation using an MTT-based colorimetric test

Samples obtained and pre-filtered according to Example 5 are analyzed using the point-of-care test strip systems described in Examples 3 and 4.

### Example 7: Clinical sample evaluation of native joints

Synovial fluid samples were retrieved from 30 clinical patients suspected of having a bacterial native joint infection. The level of D-lactate in the samples was determined as described in example 5, in each case with a filtered and a non-filtered sample, respectively. Furthermore, the samples were analyzed using cell culture, leukocyte count, and polymorphonuclear cell count. Nine of the filtered patient samples showed a level of D-lactate exceeding 0.0475 mmol/L. In six of these cases, a bacterial infection was confirmed by the cell culture results, but not in all of these cases the result would have been expected based on the measured leukocyte or polymorphonuclear cell count. In at least one further patient, a bacterial infection was suspected by the treating physician, despite cell culture did not detect a bacterial infection.

All of the cases where a bacterial infection was indeed present were successfully detected based on the level of D-lactate ([D-lactate] > 0.0475 mmol/L).

In at least one case, the leukocyte count (> 20.000/mm³) indicated a bacterial infection, although the patient did not indeed suffer from an infection, as confirmed by cell culture and D-lactate level.

The D-lactate level sample of one patient was not investigated. This patient did not suffer from a bacterial infection as determined by cell culture.

The results therefore confirmed that the level of D-lactate is a useful parameter for quickly diagnosing bacterial infections in native joints, and provides more accurate results than leukocyte count and polymorphonuclear cell count. It is therefore concluded that the diagnosis based on D-lactate allows a faster and more accurate diagnosis, and thus more effective treatment, of bacterial infections.

## Claims

1. An antimicrobial agent for use in the treatment or prevention of a bacterial infection, wherein a patient is selected for treatment using the antimicrobial agent based on the level of D-lactate in a patient sample.

2. The antimicrobial agent for use according to claim 1, wherein the patient is selected for treatment by further diagnosis of one or more further clinical parameters, wherein the further clinical parameter is preferably selected from the group consisting of cell culture, crystal formation, CRP, PCT, leukocyte count, granulocyte count, and polymorphonuclear cell count.

3. The antimicrobial agent for use according to any one of the previous claims, wherein the patient has a history of a bacterial infection or arthropathic disease, wherein said arthropathic disease is preferably a non-infectious arthritis.

4. The antimicrobial agent for use according to any one of the previous claims, wherein the patient shows ambiguous symptoms.

5. The antimicrobial agent for use according to any one of the previous claims, wherein said bacterial infection is selected from the group consisting of a joint infection, meningitis, and sepsis.

6. The antimicrobial agent for use according to claim 5, wherein said joint infection is a native joint infection or a prosthetic joint infection.

7. The antimicrobial agent for use according to any one of the previous claims, wherein said patient sample is pre-treated *in vitro* to remove contaminations before determining the level of D-lactate, wherein the sample is preferably pre-treated by filtration, centrifugation, and/or enzymatic catalysis.

8. The antimicrobial agent for use according to any one of the previous claims, wherein said patient sample is selected from the group consisting of synovial fluid, cerebrospinal fluid, and blood.

9. The antimicrobial agent for use according to any one of the previous claims, wherein determining the level of D-lactate comprises the use of an enzyme, wherein said enzyme is preferably selected from the group consisting of
(a) a protein having D-lactate oxidising activity that is independent of NAD and/or NADP,
(b) a protein having D-lactate oxidising activity isolated from a deltaproteobacterial species,
(c) a protein comprising SEQ ID NO: 1
(d) a protein comprising a sequence having at least 85%, 90% or at least 95% sequence identity to SEQ ID NO:1,
and
(e) a functional fragment of (a), (b), (c) or (d).

10. The antimicrobial agent for use according to any one of the previous claims, wherein determining the level of D-lactate is performed using a dip stick test, a flow-through test, a colorimetric test, and/or an electrochemical test.

11. The antimicrobial agent for use according to any one of the previous claims, wherein the antimicrobial agent is administered locally, preferably by irrigation.

12. The antimicrobial agent for use according to any one of the previous claims, wherein the antimicrobial agent is administered systemically, preferably intravenously or orally.

13. The antimicrobial agent for use according to any one of the previous claims, wherein the treatment further comprises a surgical intervention, preferably the mechanical removal of bacteria, a bacterial biofilm and/or infected tissue.

14. The antimicrobial agent for use according to any one of the previous claims, wherein the antimicrobial agent is administered for a duration determined by the level of D-lactate in said patient sample, preferably using repeated determination of the level of D-lactate.

15. A method for selecting a patient for treatment with an antimicrobial agent, wherein a patient is selected for treatment using the antimicrobial agent based on the level of D-lactate in a patient sample, wherein said method is preferably a computer-implemented method.
